# EUROPEAN PATENT SPECIFICATION

(11) **EP 3 798 191 B1**
(45) Date of publication and mention of the grant of the patent: **14.06.2023**
(21) Application number: 19807325.6
(22) Date of filing: 17.05.2019
(51) Int. Cl.: A61K 8/25, A61K 8/26, A61K 8/29, C09C 1/36, A61K 8/02, A61K 8/19, A61Q 1/06, A61Q 1/02, A61Q 1/08, A61Q 1/10, A61Q 1/12, A61Q 17/04, A61Q 19/00

(54) **NOVEL TITANIUM OXIDE POWDER AND COSMETIC FORMULATED THEREWITH**
NEUES TITANOXIDPULVER UND DAMIT FORMULIERTES KOSMETIKUM
NOUVELLE POUDRE D'OXYDE DE TITANE ET PRODUIT COSMÉTIQUE FORMULÉ AVEC CETTE POUDRE

(30) Priority: 23.05.2018 JP 2018099130
(43) Date of publication of application: 31.03.2021
(73) Proprietor: Kose Corporation, Tokyo 103-8251 (JP)
(72) Inventor: TOMITA, Yuriko, Tokyo 174-0051 (JP); MASUBUCHI, Yuji, Tokyo 114-0005 (JP); IDE, Miki, Tokyo 114-0005 (JP); SASAKI, Ryo, Tokyo 114-0005 (JP); KANNAN, Ryosuke, Tokyo 114-0005 (JP)
(74) Representative: D Young & Co LLP
(86) International application number: PCT/JP2019/019621
(87) International publication number: WO 2019/225491

(56) References cited:
- WO-A1-95/20184
- JP-A- H07 330 338
- JP-A- H11 157 840
- JP-A- H11 510 552
- JP-A- 2005 263 612
- JP-A- 2010 173 863

## Description

### Technical Field

The present invention relates to a novel titanium oxide powder and a cosmetic composition containing the titanium oxide powder.

### Background Art

Titanium oxide powder, which has a high refractive index, high whiteness, a high masking effect, and a high pigmenting effect, has been used as white pigments in cosmetics and paints. The titanium oxide powder, which can block ultraviolet rays, have been used as UV absorbers or UV screening agents in cosmetics (for example, see Patent Literature 1). Further applications of titanium oxide powder have been studied.

For example, white pigments composed of, for example, titanium oxide are used to hide the color unevenness, such as flecks and freckles, of skin. Unfortunately, a cosmetic containing a large proportion of titanium oxide causes opaque makeup. In order to solve this problem, Patent Literature 2 discloses a color material composition for a skin cosmetic (in claim 1) where the spectral reflectivity of the white base has a minimum value of 75% or more at a wavelength 630 to 700 nm and the white base has a Y value in a range of 25 to 65. The color material composition can provide a natural makeup texture. The patent further discloses that a foundation composition and a method of makeup can provide a natural makeup texture.

Patent Literature 3 discloses a powdered foundation composition comprising a sintered skin-color pigment that is produced by firing rutile titanium oxide with a mean particle diameter of 0.2 to 0.3 µm and an iron compound at 750 to 900°C and contains 2.9 mass% iron oxide, where the ratio a*/b* of the a* value to the b* value measured in accordance with the Hunter Lab color system is 0.24 or more and the L value is 73 or more. The foundation composition provides, for example, a natural skin tone and less color dullness due to wetting.

Patent Literature 4 discloses a cosmetic composition comprising agglomerated rutile titanium oxide particles formed by agglomeration of fan-shaped aggregations formed by aggregation and/or bonding of rod-shaped rutile titanium oxide particles each having a length of 0.05 to 0.2 µm and a width of 0.02 to 0.1 µm. The agglomerated particles each have a diameter of 0.1 to 5.0 µm and a mean coefficient of friction (µ) in the range of 0.2 or more to less than 0.7. The cosmetic composition causes, for example, no feeling of remaining on the skin.

Patent Literature 5 discloses a cosmetic composition comprising a titanium dioxide powder formed by firing titanium dioxide with acicular protrusions at 500 to 800°C. The titanium dioxide powder has an apparent mean particle diameter of 100 to 500 nm, a crystallite diameter of 15 to 30 nm measured by X-ray diffractometry, and a specific surface area of 10 to 30 m²/g. Since the disclosed cosmetic composition contains the titanium dioxide powder selectively transmitting red light, the composition can maintain a high masking effect covering, for example, flecks and freckles, diminishing the appearance of irregularities, such as pores, of the skin, and provide a natural makeup texture.

### Citation List

### Patent Literatures

Patent Literature 1: Japanese Unexamined Patent Application Publication No. Sho 62-275182
Patent Literature 2: Japanese Unexamined Patent Application Publication No. 2006-265134
Patent Literature 3: Japanese Unexamined Patent Application Publication No. 2007-145778
Patent Literature 4: Japanese Unexamined Patent Application Publication No. 2008-56535
Patent Literature 5: Japanese Unexamined Patent Application Publication No. 2017-119622

### Summary of Invention

### Technical Problem

Although users request that the skin look as natural and sharp as possible after coating of the skin with a cosmetic, the titanium oxide powders according to Patent Literatures 1 to 5 cannot achieve compatibility between the shading effect and the masking effect causing the skin to look natural, as described below in Examples.

It is a main object of the present invention to provide a titanium oxide powder that can achieve compatibility between a natural makeup texture and a shading effect to the skin after a composition containing the powder is applied onto the skin.

### Solution to Problem

The present inventors have tried a new approach to find a titanium oxide powder having optical properties that exhibit different color tones depending on view angles to achieve compatibility between a natural makeup texture and the shading effect to the skin. The present inventors have conducted extensive study and produced a novel coated titanium oxide powder including multiple coating layers composed of specific metals and having a specific mean particle diameter and undiscovered optical properties. The present inventors have found that a cosmetic or foundation composition containing the coated titanium oxide powder can provide a natural shading effect to the skin and thus completed the present invention. The present inventors have also found that the coated titanium oxide powder can reduce the pallor and yellow dullness of the skin compared to conventional titanium oxide powders.

The present inventors have accordingly completed the present invention as will be described below.

### Aspect 1

A coated titanium oxide powder including a silicon oxide or hydroxide layer, an aluminum oxide or hydroxide layer, and an iron oxide or hydroxide layer, where
the coated titanium oxide powder has a mean particle diameter D50 of 0.20 to 0.45 µm measured by analysis of a transmission electron microscopic image,
the coated titanium oxide powder has a mean particle diameter D50 of 0.6 to 1.2 µm measured by laser diffraction scattering, and
the coated titanium oxide powder in a form of a coated film has a ratio of the transmittance of transmitted light at an acceptance angle of 15 degrees to the transmittance of transmitted light at an acceptance angle of 45 degrees of 1.3 or more at a wavelength of 430 nm, and the ratio of the transmittance of transmitted light at an acceptance angle of 15 degrees to the transmittance of transmitted light at an acceptance angle of 45 degrees at a wavelength of 430 nm is equal to or higher than the ratio of the transmittance of transmitted light at an acceptance angle of 15 degrees to the transmittance of transmitted light at an acceptance angle of 45 degrees at a wavelength of 590 nm, where
the coated film is prepared by dispersing the coated titanium oxide in an amount of net 10 mass% and a copolymer of acrylate and dimethicone in an amount of 20 mass% in a volatile solvent, applying the dispersion onto a glass plate into a thickness of 25 µm, and drying the applied layer, and the transmitted light is generated by irradiation of the coated film with light from a CIE standard light source D65 of a variable angle spectrophotometric system at an incident angle of 0 degrees.

### Aspect 2

The coated titanium oxide powder described in Aspect 1, where the coated film has a maximum reflectance in a long wavelength region of 450 nm or more in a reflection spectrum at an acceptance angle of 45 degrees, where the reflected light is generated by irradiation of the coated film with light at an incident angle of 0 degrees.

### Aspect 3

The coated titanium oxide powder described in Aspect 1 or 2, where the coated film has a maximum transmittance of transmitted light in a short wavelength region of 580 nm or less in a transmission spectrum at an acceptance angle of 15 degrees and/or a transmittance of 30% or more at a wavelength of 680 nm at an acceptance angle of 15 degrees, where the transmitted light is generated by irradiation of the coated film with light at an incident angle of 0 degrees.

### Aspect 4

The coated titanium oxide powder described in any one of Aspects 1 to 3, where the coated titanium oxide powder has an aspect ratio of the major axis to the minor axis of 1.6 or less.

### Aspect 5

The coated titanium oxide powder described in any one of Aspects 1 to 4, where
the titanium oxide powder is coated with the silicon oxide or hydroxide layer, the aluminum oxide or hydroxide layer, and the iron oxide or hydroxide layer in sequence, and
the iron coating layer is an outermost, covering a surface where the silicon coating layer and the aluminum coating layer are mixed.

### Aspect 6

The coated titanium oxide powder described in any one of Aspects 1 to 5, where the surface treatment amount of aluminum is 1 to 6 mass%, the surface treatment amount of silicon oxide is 0.5 to 6 mass%, and the surface treatment amount of iron is in a co 0.5 to 3 mass%, respectively, as oxidized substance equivalent.

### Aspect 7

A cosmetic composition or a dermatological agent comprising the coated titanium oxide powder described in any one of Aspects 1 to 6.

### Aspect 8

A method for use of the coated titanium oxide powder described in any one of Aspects 1 to 6, including applying the coated titanium oxide powder to the skin to achieve a natural shading effect to the skin.

### Aspect 9

The method for use of the coated titanium oxide powder, described in Aspect 8, further including reducing pallor and yellow dullness of the skin.

The present invention provides a coated titanium oxide powder that can achieve a natural makeup texture and a shading effect to the skin after the powder is applied to the skin. The advantageous effects of the invention described in this specification should not be construed to be limiting and may be any of the advantageous effects.

### Brief Description of Drawings

Fig. 1 is a scanning electron microscopic image (photograph) of a coated titanium oxide powder (Example 2) according to an exemplary embodiment.
Fig. 2 shows transmittances at different wavelengths of a coated film in Example 1 (incident angle: 0 degrees, acceptance angle: 15, 45, and 75 degrees).
Fig. 3 shows transmittances at different wavelengths of a coated film according to Example 2 (incident angle: 0 degrees, acceptance angle: 15, 45, and 75 degrees).
Fig. 4 shows transmittances at different wavelengths of a coated film according to example 3 (incident angle: 0 degrees, acceptance angles: 15, 45, and 75 degrees).
Fig. 5 shows transmittances at different wavelengths of a coated film according to Comparative Example 1 (incident angle: 0 degrees, acceptance angles: 15, 45, and 75 degrees).
Fig. 6 shows transmittances at different wavelengths of a coated film according to Comparative Example 2 (incident angle: 0 degrees, acceptance angles: 15, 45, and 75 degrees).
Fig. 7 shows transmittances at different wavelengths of a coated film according to Comparative Example 4 (incident angle of 0 degrees, acceptance angles: 15, 45, and 75 degrees).
Fig. 8 shows reflectances at different wavelengths of coated films according to Examples 1 to 3 and Comparative Examples 1, 4, 6, 7, and 8 (incident angle: 0 degrees, acceptance angle: 45 degrees).
Fig. 9 shows transmittances at different wavelengths of coated films according to Examples 1 to 3 and Comparative Examples 1, 2, and 5 (incident angle: 0 degrees, acceptance angle: 15 degrees).
Fig. 10 shows reflectances at different wavelengths of the coated film according to Example 2 (incident angle: 0 degrees, acceptance angles: 15, 45, and 75 degrees).
Fig. 11 shows reflectances at different wavelengths of the coated film according to Comparative Example 1 (incident angle: 0 degrees, acceptance angles: 15, 45, and 75 degrees).

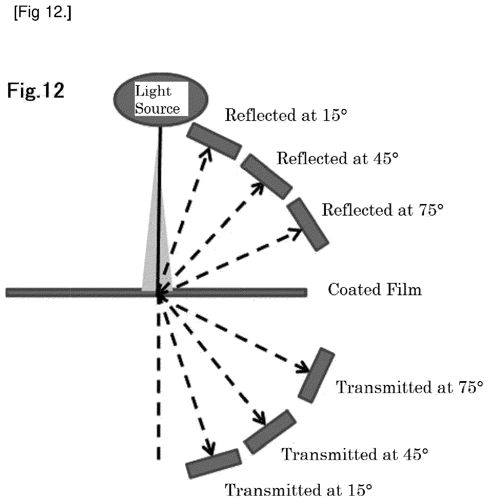

Fig. 12 is a conceptual view illustrating a variable angle spectrophotometric system including a light source, reflected light, transmitted light, and a coated film. The drawing exemplifies light reflected and received at 15, 45, and 75 degrees. It should be noted that the angles measureable by the variable angle spectrophotometric system is also applicable to any acceptance angle

### Description of Embodiments

Preferred embodiments of the present invention will now be described in detail, with the proviso that the invention may include any other embodiment modified within the scope of the invention. The indication of percentage in this specification refers to that by mass, unless otherwise described. The numerical range representing with "to" in this specification is to include the numerical values at both ends. The upper limits and lower limits of numerical ranges can be appropriately combined as desired.

### 1. Coated Titanium Oxide Powder

A coated titanium oxide powder according to the present invention will now be described in detail in Sections (a) Core and Coating Layer of Inventive Coated Titanium Oxide Powder, (b) Mean Particle Diameter D50, Measured by Image Analysis, of Inventive Coated Titanium Oxide Powder, (c) Mean Particle Diameter D50, Measured by Laser Diffraction Scattering, of Inventive Coated Titanium Oxide Powder, (d) Isoelectric Point of Inventive Coated Titanium Oxide Powder, and (e) Optical Properties of Inventive Coated Titanium Oxide Powder. The following description, however, should not be construed to limit the coated titanium oxide powder of the present invention.

The coated titanium oxide powder according to the present invention preferably satisfies at least one of the following conditions (a), (b), (c), and (e), and more preferably the optional condition (d):
(a) the coated titanium oxide powder includes a silicon oxide or hydroxide, an aluminum oxide or hydroxide, and an iron oxide or hydroxide, respectively, as each layer;
(b) the coated titanium oxide powder has a mean particle diameter D50 of 0.20 to 0.45 µm measured by analysis of a transmission electron microscopic image;
(c) the coated titanium oxide powder has a mean particle diameter D50 of 0.6 to 1.2 µm measured by laser diffraction scattering;
(d) the coated titanium oxide powder has an isoelectric point ranging from 1.5 to 6.0; and
(e) the coated film of the coated titanium oxide has distinguishing optical properties measured by a variable angle spectrophotometric system.

In condition (e), it is preferred that the coated titanium oxide powder in a form of a coated film composed of the coated titanium oxide and an copolymer of acrylate and dimethicone has a ratio of the transmittance of transmitted light at an acceptance angle of 15 degrees to the transmittance of transmitted light at an acceptance angle of 45 degrees of 1.3 or more at a wavelength of 430 nm and/or the ratio of the transmittance of the transmitted light at an acceptance angle of 15 degrees to the transmittance of the transmitted light at an acceptance angle of 45 degrees is equal to or higher than that at a wavelength of 590 nm, where the transmitted light is generated by irradiation of the coated film with light from a CIE standard light source D65 of a variable angle spectrophotometric system at an incident angle of 0 degrees.

It is more preferred that the coated titanium oxide powder has a ratio of the transmittance of the transmitted light at an acceptance angle of 15 degrees to the transmittance of the transmitted light at an acceptance angle of 45 degrees is 1.3 or more and that the ratio of the transmittance of transmitted light at an acceptance angle of 15 degrees to the transmittance of transmitted light at an acceptance angle of 45 degrees is equal to or higher than that at an wavelength of 590 nm.

The coated film is prepared by dispersing the coated titanium oxide in an amount of 10 mass% and a copolymer of acrylate and dimethicone in an amount of 20 mass% in a volatile solvent (the balance; for example, isopropyl alcohol), applying the dispersion onto a glass plate into a thickness of 25 µm, and drying the applied layer.

The coated titanium oxide powder according to the present invention has a novel morphology and optical properties. Coating of the skin with the coated titanium oxide powder according to the invention can provide a natural shading effect to the skin with reduced pallor and yellow dullness.

Common titanium oxide has optical properties that significantly affect cosmetic films. When common titanium oxide dominantly reflects bluish light, the face looks pale, resulting in a negative impression. When titanium oxide dominantly reflects yellowish light, the face looks dull. Thus, adjustment of the color by reflection of light tends to result in unnatural appearance of the face. In conclusion, common titanium oxide precludes a balanced appearance of the face.

In contrast, the coated titanium oxide powder according to the present invention has the following advantageous properties: First, the reflection of purple light is not significantly high after coating of the skin with the powder. The second, reflected light is not completely white because the powder has an uneven reflection spectrum (different intensities having peaks and bottoms at different wavelengths) over the entire visible region; hence, the powder does not exhibit odd white undertone nor emphasize specific colors by reflection. Third, the titanium oxide powder according to the present invention transmits clear purple-bluish light from the skin without excess masking falling in front of the face but warm reddish light (less pallid) from the cheek skin with a natural shading effect on both sides of the face. This is because the coated titanium oxide powder according to the present invention can selectively transmit substantially orthogonal light, predominantly transmit purple-bluish light to shade yellow dullness of the skin, and moderately transmit substantially direct light to exhibit a reddish appearance, resulting in a shading effect and a natural masking feeling. The coated titanium oxide powder of the invention can accordingly take advantage of the internal light from the skin after it is applied onto the skin.

### (a) Core and Coating Layer of Inventive Coated Titanium Oxide Powder

The coated titanium oxide powder according to the present invention includes a silicon oxide or hydroxide, an aluminum oxide or hydroxide, and an iron oxide or hydroxide, respectively, as each layer under condition (a).

The core composed of a titanium oxide particle is preferably coated with, in sequence, silicon oxide or hydroxide, aluminum oxide or hydroxide, and iron oxide or hydroxide (the resulting layers are also referred to as "silicon coating layer", "aluminum coating layer", and "iron coating layer").

The core, which is composed of a titanium oxide particle according to the present invention, is coated with silicon oxide or hydroxide. The silicon coating layer is incompletely coated with aluminum oxide or hydroxide. The particle of the titanium oxide according to the invention, which is further provided with the silicon coating layer and the incomplete aluminum coating layer, is coated with iron oxide or hydroxide; hence, the iron coating layer is the outermost layer in the titanium oxide powder of the invention.

The coated titanium oxide powder according to the present invention, which includes multiple coating layers, the incomplete aluminum coating layer and the outermost iron coating layer, can more effectively provide a natural shading effect to the skin with reduced pallor and yellow dullness. The titanium oxide powder according to the invention, which includes the coating layers, can be readily spread on the skin and have a satisfactory masking effect.

The titanium oxide powder according to the present invention is preferably covered such that metal components have the following specific ranges of contents. Such ranges of contents can more significantly provide a natural shading effect to the skin with reduced pallor and yellow dullness:
The surface treatment amount of aluminum in the titanium oxide powder of the present invention is preferably 1 to 6 mass%, more preferably 1 to 5 mass%, further preferably 2 to 5 mass%, as oxidized substance equivalent.

The surface treatment amount of silicon in the titanium oxide powder of the invention is preferably 0.5 to 6 mass%, more preferably 1 to 6 mass%, further preferably 3 to 6 mass%, as oxidized substance equivalent.

The surface treatment amount of iron in the titanium oxide powder of the invention is preferably 0.5 to 3 mass%, more preferably 0.5 to 2 mass%, further preferably 0.5 to 1.5 mass%, as oxidized substance equivalent. The outermost iron coating layer disposed such an iron oxide content can prevent the powder according to the invention from exhibiting a pale color tone and excess yellow dullness.

It is more preferred to adjust the contents of the metal oxides on the titanium oxide powder of the invention such that the surface treatment amount of aluminum is 1 to 6 mass%, the surface treatment amount of silicon is 0.5 to 6 mass%, and the surface treatment amount of iron oxide is in 0.5 to 3 mass%, respectively, as oxidized substance amount equivalent.

### Measurement of Metal Contents after Surface Treatment

The metal contents after the surface treatment in a sample (powder) prepared by a common technique can be determined with an inductively coupled plasma (ICP) atomic emission spectroscope (ARCOS^{®} available from SPECTRO in Germany).

The titanium oxide powder of the present invention (also referred to as "titanium oxide core") before coating can be prepared by a known technique for production of titanium oxide powder. Alternatively, the core may be a commercially available product (for example, see Patent Literature 1). The titanium oxide powder can be appropriately selected. For example, core particles can be selected that can produce a coated titanium oxide powder with a mean particle diameter measured by analysis of a transmission electron microscopic image in Section (b) and by laser diffraction scattering in Section (c), which will be described below.

Titanium oxide of the core are present in both an anatase crystal form and a rutile crystal form, a core with the rutile crystal form is preferred because it has a low optical catalytic activity, a high refractive index, and thus a high masking effect. In order to stabilize the crystal, the core of the titanium oxide powder preferably contain a small amount of zinc oxide. The content of the zinc oxide in the titanium oxide core is preferably 1 mass% or less, more preferably 0.1 to 0.8 mass%, further preferably 0.2 to 0.7 mass%.

The core of the titanium oxide may have any shape, for example, a straw-bundle-like, strip-like, spherical, acicular, or rod-like shape. In particular, the titanium oxide used as the core of the present invention preferably has a spherical shape with a relatively smooth surface rather than the shape of marimo (round green alga) or a chestnut burr, having a large number of acicular protrusions.

Coating with silicon oxide or hydroxide can be carried out by any known technique. Examples of the technique include adding sodium metasilicate or an aqueous sodium metasilicate solution to an aqueous slurry containing powder particles and then adding acid, for example, sulfuric acid to the aqueous slurry to precipitate silica or its hydrate; and dispersing the powder particles in a solvent, such as alcohol, water, or mixture of alcohol and water and adding an alkoxysilane-based metal coupling agent, such as tetraethoxysilane, and an acid or base to the solvent to precipitate silicon or its hydroxide by heating. Subsequent washing, filtering, and drying to produce coated particles.

Coating with aluminum oxide or hydroxide may be carried out by any known technique. An example of such a technique includes adding, for example, aluminum nitrate, aluminum sulfate, or sodium aluminate or an aqueous solution thereof to an aqueous slurry containing powder particles and then adding acid or alkali to precipitate an aluminum compound. Another example of the technique includes dispersing the powder particles in a solvent, such as alcohol, water, or a mixture of alcohol and water and adding an aluminum-based metal coupling agent and an acid or base to precipitate aluminum or its hydroxide by heating. Subsequent washing, filtering, and drying to produce coated particles.

Iron oxide or hydroxide can be applied by any known technique. For example, an aqueous sodium hydroxide solution is heated to 30 to 35°C such that the powder particles are dispersed. An aqueous ferric sulfate solution is added and stirred at 90°C for 2 hours to complete the reaction. The resultant complex compound is washed with water, neutralized, and then dried. The complex compound is pulverized and then fired at, for example, about 750 to 900°C for about 1 to 3 hours, resulting in oxide or hydroxide of the powder particles and iron.

Examples of the iron compound include ferric oxide, ferrous oxide, iron(II,III) oxide, iron hydroxide, organic acid salts of iron, such as iron oxalate and iron citrate, inorganic acid salts of iron, such as iron chloride, ferric sulfate, and iron sulfate. One or more iron compounds can be selected from this group. The iron compound, i.e., the iron oxide or hydroxide is present on or near the surfaces of the particles.

### (b) Mean Particle Diameter D50 Measured by Image Analysis of Inventive Coated Titanium Oxide Powder

The coated titanium oxide powder according to the present invention has a lower limit of a mean particle diameter D50 of preferably 0.10 µm, more preferably 0.20 µm, further preferably 0.25 µm, and an upper limit of the mean particle diameter D50 of preferably 0.60 µm, more preferably 0.50 µm, further preferably 0.45 µm, still further preferably 0.40 µm, as measured by analysis of a transmission electron microscopic image. In consequence, the mean particle diameter D50 is more preferably in a range of 0.20 to 0.45 µm, further preferably 0.25 to 0.45 µm.

The coated titanium oxide powder having a mean particle diameter in such a range can be readily spread over the skin and have a satisfactory masking effect. A more preferred natural shading effect can thereby be provided to the skin with reduced pallor and yellow dullness.

The coated titanium oxide powder has an upper limit of the aspect ratio (major axis/minor axis) of preferably 1.7, more preferably 1.6, and a lower limit of the aspect ratio of preferably 1.2, more preferably 1.3, further preferably 1.4. In consequence, it is more preferred that the coated titanium oxide powder has an aspect ratio in a range of more preferably 1.3 to 1.7.

The coated titanium oxide powder having an aspect ratio in such a range can be readily spread over the skin and have a satisfactory masking effect. A more preferred natural shading effect can thereby be provided to the skin with reduced pallor and yellow dullness.

### Measurement of Mean Particle Diameter D50 and Aspect Ratio by analysis of Transmission Electron Microscopic Image

About 10 mg of the coated titanium oxide powder is thoroughly kneaded with to be dispersed with 1-propanol, and is spread over a glass slide into a thin film sample. The thin film sample is placed on a mesh laminated with a support film for measurement with a transmission electron microscope (TEM). The sample on the mesh (dry film) is placed on a transmission electron microscope system (S-4800 available from Hitachi High-Technologies) to capture images of individual particles on the dried coated film. Each image of about 1000 particles on the dried film are processed with image analyzing particle size distribution measurement software (Mac-View available from MOUNTECH Co., Ltd.) to determine the particle diameters. In this way, the mean particle diameter D50 of the sample (powder) can be determined by analysis of transmission electron microscopic (TEM) images. The aspect ratio (major axis/minor axis) can be also determined by analysis of the transmission electron microscopic images.

### (c) Mean Particle Diameter D50, Measured by Laser Diffraction Scattering, of Inventive Coated Titanium Oxide Powder

The coated titanium oxide powder according to the present invention has a lower limit of the mean particle diameter D50 of preferably 0.50 µm, more preferably 0.6 µm, further preferably 0.7 µm, still further preferably 0.8 µm, more preferably 0.9 µm, and an upper limit of the mean particle diameter D50 of preferably 1.5 µm, more preferably 1.4 µm, further preferably 1.2 µm, more preferably 1.2 µm, more preferably 1.1 µm, as measured by laser diffraction scattering. In consequence, the coated titanium oxide powder has a mean particle diameter D50 in a range of more preferably 0.6 to 1.2 µm, further preferably 0.8 to 1.1 µm.

The coated titanium oxide powder having a particle diameter in such a range can be readily spread over the skin and have a satisfactory masking effect. A more preferred natural shading effect can thereby be provided to the skin with reduced pallor and yellow dullness.

### Mean Particle Diameter D50 measured by Laser Diffraction Scattering

The sample (powder) is subjected to ultrasonic vibration in a methanol medium for 60 seconds and then poured over a slot of a laser diffraction scattering particle size distribution analyzer (Microtrac HRA available from NIKKISO CO., LTD.). In this way, the mean particle diameter D50 (median diameter) of the sample (powder) can be determined by laser diffraction scattering.

### (d) Isoelectric Point of Inventive Coated Titanium Oxide Powder

The coated titanium oxide powder according to the present invention may have any isoelectric point. The lower limit is preferably 1.5, more preferably 2.0, whereas the upper limit is preferably 6.0, more preferably 5, further preferably 4.0. In consequence, the isoelectric point is in a range of more preferably 3 ± 1.0. The isoelectric point can be determined by measurement of the zeta potential in a dispersion of the coated titanium oxide powder, for example, by the test method of the isoelectric point of fine ceramic powder in accordance with JIS R 1638:1999.

The coated titanium oxide having an isoelectric point in such a range can have high dispersion stability in both the powder and liquid formulations, and thus can be readily spread over the skin and have a satisfactory masking effect. A more satisfactory natural shading effect can thereby be provided to the skin with reduced pallor and yellow dullness.

### (e) Optical Properties of Inventive Coated Titanium Oxide Powder

The coated film prepared with the coated titanium oxide powder according to the present invention has characteristic optical properties measured with a variable angle spectrophotometric system as required in condition (e). As described in Section "Measurement with Variable Angle Spectrophotometric System" below, the optical properties can be determined by measurement of the coated film of the invention.

It is preferred that the coated titanium oxide powder of the invention has at least one of the optical properties selected from conditions (e1) to (e5), which will be described below.

The coated titanium oxide powder according to the present invention preferably has optical properties required at least one of in condition (e1) the ratio of the transmittance at an acceptance angle of 15 degrees to the transmittance at an acceptance angle of 45 degrees at a wavelength of 430 nm and/or condition (e2) the ratio of the transmittance at an acceptance angle of 15 degrees to the transmittance at an acceptance angle of 45 degrees at a wavelength of 430 nm> the ratio of the transmittance at an acceptance angle of 15 degrees to the transmittance at an acceptance angle of 45 degrees at a wavelength of 590 nm. It is more preferred that the coated titanium oxide powder of the invention has the optical properties under both of conditions (e1) and (e2).

It is preferred that the coated titanium oxide powder according to the present invention has at least one of the optical properties under conditions: (e3) the wavelength at a maximum reflectance in a reflection spectrum, (e4) the transmittance at an acceptance angle of 15 degrees and a wavelength of 680 nm, and (e5) the wavelength at a maximum transmittance in a transmission spectrum, in addition to conditions (e1) and (e2).

Examples of the preferred combination of conditions (e1) and (e2) with conditions (e3) to (e5) include: combination of conditions (e1) and (e2) with condition (e3); combination of conditions (e1) and (e2) with condition (e4); combination of conditions (e1) and (e2) with condition (e5). Such combinations may be further combined with any other condition of optical properties.

Examples of the preferred combination of conditions (e3) to (e5) include: combination of conditions (e3) and (e4); combination of conditions (e3) and (e5); combination of conditions (e4) and (e5); and combination of conditions (e3), (e4), and (e5). Combination of conditions (e3), (e4), and (e5) is more preferred.

### (e1) Ratio of Transmittance at 15 Degrees to Transmittance at 45 Degrees at Wavelength of 430 nm

(e1) The coated titanium oxide powder in a form of a coated film has a ratio of the transmittance of transmitted light at an acceptance angle of 15 degrees to the transmittance of transmitted light at an acceptance angle of 45 degrees of preferably 1.3 or more at a wavelength of 430 nm, where the transmitted light is generated by irradiation of the coated film with light from a CIE standard light source D65 of a variable angle spectrophotometric system at an incident angle of 0 degrees.

The ratio of the transmittance at an acceptance angle of 15 degrees to the transmittance at an acceptance angle of 45 degrees at a wavelength of 430 nm in condition (e1) has a lower limit of preferably 1.3, more preferably 1.4, further preferably 1.5, and an upper limit of preferably 2.5, more preferably 2.3, further preferably 2.1, still further preferably 1.9. In consequence, the ratio is in a range of preferably 1.4 to 2.3, more preferably 1.4 to 1.9.

The advantageous effects of the present invention can thereby be more effectively achieved from the viewpoint of a natural makeup texture and shading effect to the skin with reduced pallor and yellow dullness.

### (e2) Ratio of Transmittance at 15 Degrees to Transmittance at 45 Degrees at Wavelength of 430 nm ≥ Ratio of Transmittance at 15 Degrees to Transmittance at 45 Degrees at Wavelength of 590 nm

Under condition (e2), it is preferred that the ratio of the transmittance of the transmitted light at an acceptance angle of 15 degrees to the transmittance of the transmitted light at an angle of 45 degrees at a wavelength of 430 nm is equal to or higher than the ratio of the transmittance of the transmitted light at an acceptance angle of 15 degrees to the transmittance of the transmitted light at an acceptance angle of 45 degrees at a wavelength of 590 nm, where the transmitted light is generated by irradiation of the coated film with light from the CIE standard light source D65 of the variable angle spectrophotometric system at an incident angle of 0 degrees.
the ratio of the transmittance of the transmitted light at an acceptance angle of 15 degrees to the transmittance of the transmitted light at an angle of 45 degrees at a wavelength of 430 nm under condition (e2) is preferably equal to or greater than the ratio of the transmittance of the transmitted light at an acceptance angle of 15 degrees to the transmittance of the transmitted light at an acceptance angle of 45 degrees at a wavelength of 590 nm.

The ratio of the transmittance ratio at an acceptance angles of 15 and 45 degrees at a wavelength of 430 nm to the transmittance ratio at an acceptance angles of 15 and 45 degrees at a wavelength of 590 nm is preferably 2.0 or less, more preferably 1.8 or less, further preferably 1.5 or less, still further preferably 1.4 or less, more preferably 1.3 or less, under condition (e2).

The advantageous effects of the present invention can thereby be more effectively achieved from the viewpoint of a natural makeup texture and shading effect to the skin with reduced pallor and yellow dullness.

### (e3) Wavelength at Maximum Reflectance in Reflection Spectrum

Under condition (e3), the light, which is radiated to the coated film from the CIE standard light source D65 of the variable angle spectrophotometric system at an incident angle of 0 degrees and reflected at an acceptance angle of 45 degrees, preferably has a maximum reflectance in a long wavelength region of 450 nm or more. The maximum reflectance refers to the highest reflectance within a reflection spectrum.

The wavelength of the maximum reflectance under condition (e3) has a lower limit of preferably 450 nm, more preferably 480 nm, further preferably 490 nm, still further preferably 500 nm, still further preferably 510 nm, and an upper limit of preferably 700 nm, more preferably 650 nm, further preferably 590 nm, still further preferably 570 nm, more preferably 550 nm. More preferably, the maximum reflectance under condition (e3) is in a range of 480 to 580 nm.

The advantageous effects of the present invention can thereby be more effectively achieved from the viewpoint of a natural makeup texture of the skin with reduced pallor and yellow dullness.

### (e4) Transmittance at 15 Degrees and 680 nm

Under condition (e4), the light, which is radiated to the coated film at an incident angle of 0 degrees and transmitted at an acceptance angle of 15 degrees, preferably has a transmittance of 30% or more at a wavelength of 680 nm.

The transmittance is preferably 30% or more, more preferably 33% or more, further preferably 35% or more, at an acceptance angle of 15 degrees and a wavelength of 680 nm. The transmittance is preferably as high as possible and thus has an upper limit of, for example, about 98%. The advantageous effects of the present invention can thereby be more effectively achieved from the viewpoint of a natural makeup texture and shading effect to the skin with reduced pallor and yellow dullness.

### (e5) Wavelength at Maximum Transmittance in Transmission Spectrum

Under condition (e5), it is more preferred that the light, which is radiated to the coated film at an incident angle of 0 degrees and transmitted at an acceptance angle of 15 degrees, has a maximum transmittance at a short wavelength region of 580 nm or less in a transmission spectrum. The maximum transmittance refers to the highest transmittance in the transmission spectrum.

The wavelength of the maximum transmittance under condition (e5) has an upper limit of preferably 670 nm, more preferably 600 nm, further preferably 580 nm, and a lower limit of preferably 440 nm, more preferably 460 nm, further preferably 480 nm. More preferably, the wavelength of the maximum transmittance under condition (e5) is in a range of 480 to 580 nm.

The advantageous effects of the present invention can thereby be more effectively achieved from the viewpoint of a natural makeup texture of the skin with reduced pallor and yellow dullness.

Preferably, the coated titanium oxide powder according to the present invention has at least one of the optical properties under conditions (e1) to (e5) and further has at least one of the optical properties under conditions: (e6) the ratio of the reflectance at an acceptance angle of 45 degrees and a wavelength of 450 nm to the reflectance at an acceptance angle of 45 degrees and a wavelength of 650 nm; (e7) the ratio of transmittance at a wavelength of 430 nm (at acceptance angles of 45 and 75 degrees), and the ratio of transmittance at a wavelength of 590 nm (at acceptance angles of 45 and 75 degrees) ; and (e8) the ratio of the reflectance at an acceptance angle of 15 degrees to the reflectance at an acceptance angle of 45 degrees at a wavelength of 450 nm, and the reflectance at an acceptance angle of 45 degrees to the reflectance at an acceptance angle of 75 degrees at a wavelength of 650 nm.

### (e6) Ratio of Reflectance at 45 Degrees and 450 nm to Reflectance at 45 Degrees and 650 nm

Under condition (e6), the ratio of the reflectance of the light at an acceptance angle of 45 degrees and wavelength of 450 nm to the reflectance of the light at an acceptance angle of 45 degrees and a wavelength of 650 nm has a lower limit of preferably 0.5, more preferably 0.6, further preferably 0.7, still further preferably 0.8, and an upper limit of preferably 1.3, more preferably 1.2, further preferably 1.1, where the transmitted light is generated by irradiation of the coated film with light from the CIE standard light source D65 of the variable angle spectrophotometric system at an incident angle of 0 degrees.

The advantageous effects of the present invention can thereby be more effectively achieved from the viewpoint of a natural makeup texture and shading effect to the skin with reduced pallor and yellow dullness.

### (e7) Ratio of Transmittance at a wavelength of 430 nm (at acceptance angles of 45 and 75 degrees) and ratio of Transmittance at a wavelength of 590 nm (at acceptance angles of 45 and 75 degrees)

Under condition (e7), the ratio of the transmittance of the transmitted light at an acceptance angle of 45 degrees to the transmission of the transmitted light at an acceptance angle of 75 degrees is preferably ±10%, more preferably ±5%, further preferably ±3%, at a wavelength of 430 nm, where the transmitted light is generated by irradiation of the coated film with light at an incident angle of 0 degrees.

The ratio of the transmittance of the transmitted light at an acceptance angle of 45 degrees to the transmittance of the transmitted light at an acceptance angle of 75 degrees is preferably ±10%, more preferably ±5%, further preferably ±3%, at a wavelength of 590 nm, where the transmitted light is generated by irradiation of the coated film with light at an incident angle of 0 degrees.

More preferably, both the transmittance ratio at a wavelength of 430 nm (at acceptance angles of 45 and 75 degrees) and the transmittance ratio at a wavelength of 590 nm (at acceptance angles of 45 and 75 degrees) lie within the ranges described above.

The advantageous effects of the present invention can thereby be more effectively achieved from the viewpoint of a natural makeup texture and shading effect to the skin with reduced pallor and yellow dullness.

Under condition (e8), (i) the ratio of the reflectance of reflected light at an acceptance angle of 15 degrees to the reflectance of reflected light at an acceptance angle of 45 degrees at a wavelength of 450 nm and/or (ii) the reflectance of reflected light at an acceptance angle of 45 degrees to the reflectance of reflected light at an acceptance angle of 75 degrees at a wavelength of 650 nm is preferably within ±10%, where the reflected light is generated by irradiation of the coated film with light at an incident angle of 0 degrees. Such a ratio of the reflectance is preferred, for example, from the viewpoint of natural makeup texture and shading effect to the skin.

### Measurement with Variable Angle Spectrophotometric System

The spectrophotometric color difference to a sample is measured with a variable angle spectrophotometric system GSP-01B available from Murakami Color Research Laboratory. The glass plate has a thickness of 3 mm and the distance from the CIE standard D65 halogen light source to the coated film is about 30 cm. The light is radiated from the CIE standard light source to the coated film at an incident angle of 0 degrees to determine measurements at acceptance angles of 0 to 90 degrees (in particular, 15, 45, and 75 degrees) and transmission angles of 0 to 90 degrees (in particular, 15, 45, and 75 degrees). The optical properties of the sample (powder) can thereby be determined.

The coated film can be prepared as follows: A mixture of 10% sample (power: pure coated titanium oxide or pure titanium oxide) and 20% copolymer of acrylate and dimethicone is forcibly dispersed in a volatile solvent (isopropyl alcohol (IPA)) with the aid of zirconia beads. The dispersion is applied onto the glass plate with a doctor blade into a thickness of 25 µm and then the coating is dried to form a resin coated film for measurement of a color difference with the variable angle spectrophotometric system. The optical properties of the sample (the powder) contained in the coated film are determined.

The common titanium oxide powder cannot achieve a natural shading effect to the skin. The common titanium oxide powder tends to exhibit the pallor and/or the yellow dullness of the skin.

For example, Patent Literatures 2 and 5 each disclose technology of a low transmittance at a wavelength of 630 to 700 nm indicating transmission of red light. As Patent Literatures 2 and 5 disclose that the reflection of the light at a wavelength of 450 nm is 1.3 times that at a wavelength of 650 nm, the reflection of the blue to green light region is significantly high, resulting in unnatural whiteness due to the reflection properties in many cases.

For example, Patent Literature 4 discloses that the color tone depends on the particle diameter of the powder. Powder having a particle diameter of less than 1 µm exhibit unnatural pallor similar to that disclosed in Patent Literature 5. Powder having a particle diameter of about 1 µm have a preferred color tone but fall selectively into pores of the skin to cause uneven color tone. Powders having a larger particle diameter evenly reflects the entire visible light region to cause the skin to look white. Thus the skin tends to look unnaturally white although the masking effect is reduced.

For example, Patent Literature 3 discloses spherical titanium oxide complexed with iron oxide. Although this technology relates to reflection and transmission that emphasize the reddishness, the reflection by the iron oxide cannot avoid an increase in yellow dullness. As a result, the yellow dullness of the skin tends to be emphasized. Patent Literature 5 discloses a core of titanium oxide having a shape of marimo (round green alga) with acicular protrusions. Iron oxide is sintered on the core, nevertheless the original intense pallor of the core cannot be completely wiped out, precluding the intended improvement in some cases.

In contrast, the coated titanium oxide powder according to the present invention can achieve a natural shading effect to the skin after it is applied to the skin, as described in Examples below. In addition, the present invention can reduce the pallor and yellow dullness of the skin. The novel powder of the present invention accordingly has a morphology and properties incomparable to conventional titanium oxide powders.

The coated titanium oxide powder according to the present invention, which has a specific shape and morphology, has high dispersibility that can achieve a natural makeup that provides a fresh feeling of bare skin to everyone.

The coated titanium oxide powder of the invention can achieve a natural covering effect that readily transmits direct light, in other words, mainly transmits white light without yellow dullness from the interior of the skin and provides natural masking feeling. The coated titanium oxide powder of the invention can maintain an appropriate natural covering effect (the so-called masking effect) that can be partially adjusted by users. Conventional titanium oxide powder causes so-called unnatural whiteness due to much foundation or an insufficient masking effect due to less foundation to prevent unnatural whiteness. In contrast, the coated titanium oxide powder of the invention enables a user to facilitate a natural makeup texture and shading effect to the skin.

The coated titanium oxide powder according to the present invention can achieve a natural shading effect to the skin with reduced pallor and/or yellow dullness after it is applied to the skin. The titanium oxide powder of the invention can be used on the skin, and applied to the skin, for example, by coating or spraying in expectation of such effects, and can be used in makeup and other methods. In such application, coating is preferred because a natural makeup texture and shading effect to the skin can be readily achieved.

The coated titanium oxide powder of the present invention can be surface-treated within the scope that can maintain the advantageous effects of the invention. The surface treatment of the coated titanium oxide powder of the invention can improve usability and lasting of the makeup due to, for example, the high viscosity, the dispersibility into oil, and the water repellency of the powder. The powder can be surface-treated by any known method.

A water-repelling and/or oleophobic surface treatment of the coated titanium oxide powder can be carried out with, for example, a silicone compound, such as poly(dimethyl siloxane) and poly(methyl hydrogen siloxane); a coupling agent, such as silane coupling agent for a treatment with alkyl alkoxysilane, aluminum coupling agent, or titanium coupling agent; a fluorine compound, such as perfluoroalkyl alkoxysilane; hydrocarbon; lecithin or hydrogenated lecithin; amino acid or peptide; an amino acid derivative, such as N-acylamino acid, e.g., lauroyl lysine, sodium dilauroyl glutamic lysine, disodium stearoyl glutamate, or sodium lauroyl aspartate; polyethylene; a wax; a fatty acid, such as stearic acid, palmitic acid, or myristic acid, fatty acid salt, fatty acid ester, or fatty acid amide; or a metal soap.

The coated titanium oxide powder according to the present invention can be compounded into, for example, cosmetics, dermatological agents, and pigments. Among these applications are preferred cosmetics and dermatological agents that can impart a natural makeup texture and shading effect to the skin.

A cosmetic or dermatological agent according to the present invention may have any dosage form, for example, powder, solidified powder, cream, emulsion, lotion, oily liquid, oily solid, paste, or spray solution. Before being compounded into the cosmetic or dermatological agent of the invention, the coated titanium oxide powder may be preliminarily complexed with any other powder. The coated titanium oxide powder may be complexed by any technique and have any shape and particle diameter distribution after the complexation. The coated titanium oxide powder of the invention may be compounded in any content, preferably 0.01 to 40 mass%, more preferably 0.1 to 30 mass%.

The cosmetic or dermatological agent according to the present invention may contain any other component within the range of the advantageous effects of the invention. Examples of such a component include oily components, dyes, pH adjusters, humectants, thickeners, surfactants, dispersants, stabilizers, colorants, antiseptics, antioxidants, sequestrants, astringents, antiphlogistics, UV absorbers, fragrances, and other pigments. Examples of the pigment and UV absorber include particles of iron oxide indices, such as red iron oxide, yellow iron oxide, and black iron oxide, which have high refractive indices; zinc oxide particles; and titanium oxide particles other than that of the present invention. The pigments and UV absorbers may have any size and shape (specifically, a mean particle diameter of 0.01 to 10 µm) that can block the light in a near-infrared region, visible region, and/or ultraviolet region within the range of the advantageous effect of the invention.

Examples of the application of the cosmetics and dermatological agent containing the coated titanium oxide powder according to the present invention include makeup cosmetics, such as loose face powder or oshiroi (traditional Japanese white powder foundation), makeup base materials, foundation compositions, concealers, face powders, color controller, sunscreen cosmetic compositions, lip rouges, lip creams, eye shadows, eye liners, mascaras, blushers, manicure solutions, body powders, perfume powders, and baby powders; basic skin care products, such as emulsions, creams, and sunscreens; hair cosmetics; body care cosmetics; skin care cosmetics; and hair care cosmetics. Particularly preferred are cosmetics and dermatological agents, such as oshiroi, bases, foundations, concealers, face powders, control colors, sunscreen cosmetics, and body powders to achieve a natural shading effect to the skin. More preferred are cosmetics and dermatological agents applicable to the face to achieve a natural shading effect to the skin.

### Examples

The present technique will now be described in more detail by way of the following Examples and Comparative Examples which should not be construed to limit the present invention.

Titanium dioxide (100 g) was placed in a quartz crucible. The titanium dioxide in the crucible was fired at 500 to 900°C for 1 hour in a muffle furnace, resulting in rutile titanium dioxide powder as a core. The titanium dioxide powder core had a mean particle diameter of about 0.2 to 0.3 µm determined by analysis of transmission electron microscopic images, and a spherical shape without hairy acicular protrusions.

An aqueous slurry containing the rutile titanium oxide powder core was prepared. An aqueous solution containing hydrous silica and then acid were added to the slurry so as to precipitate a hydrous silica compound. The slurry was dried and pulverized into a hydrous-silica-coated titanium oxide powder. An aqueous slurry containing the hydrous-silica-coated titanium oxide powder was prepared. An aqueous aluminum hydroxide solution was added to the slurry. Acid was then added to partially precipitate an aluminum hydroxide compound to form incomplete coating. The slurry was then dried and the dried product was pulverized.

The titanium oxide powder, which has been coated with hydrous silica and then aluminum hydroxide, was further coated with iron oxide. The titanium oxide powder with an incomplete aluminum hydroxide coating layer was added to an aqueous ferric sulfate solution. The solution was stirred at 90°C for about 2 hours to complete the reaction. The resultant complex was washed with water, neutralized, and then dried. After being pulverized, the complex was fired at 750 to 900°C for 2 to 3 hours in an electric furnace. The fired complex was then screened to produce each of the coated titanium oxide powders according to Examples 1 to 3.

As shown in Table 1, the titanium oxide powders according to Comparative Examples 1 to 8 were prepared by appropriately modifying coating layers or commercially available products.

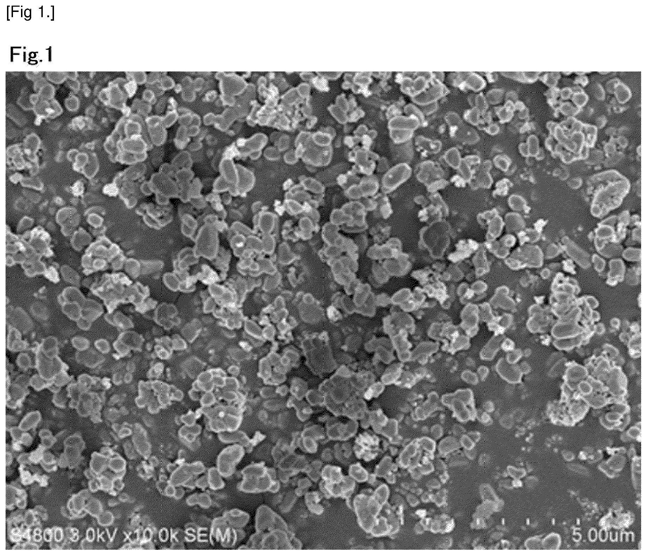

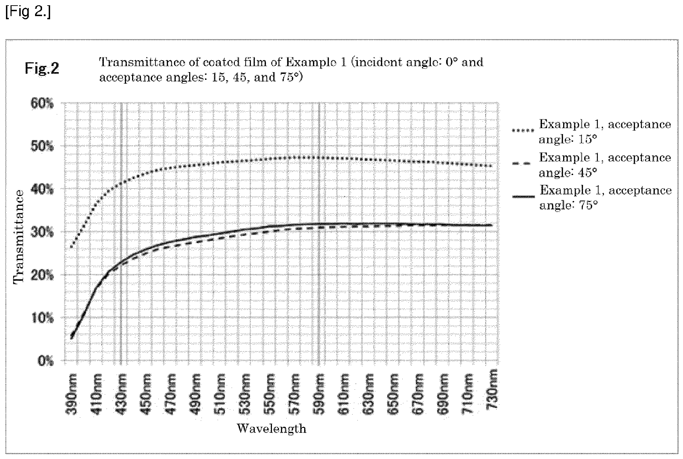

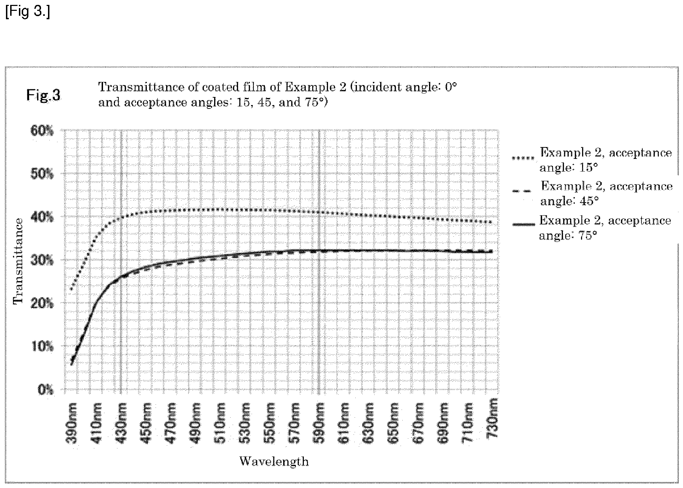

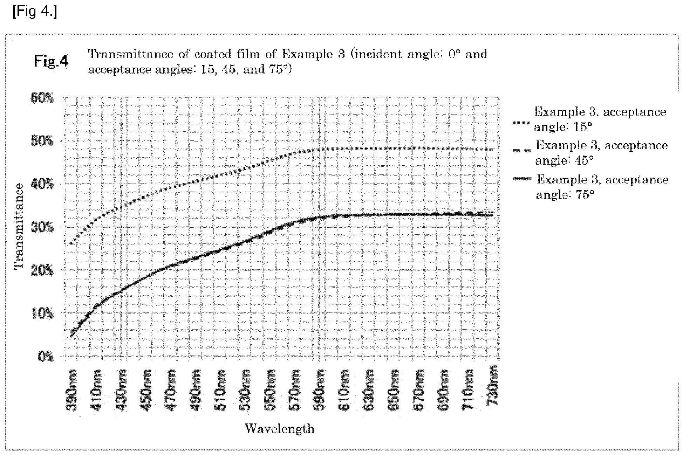

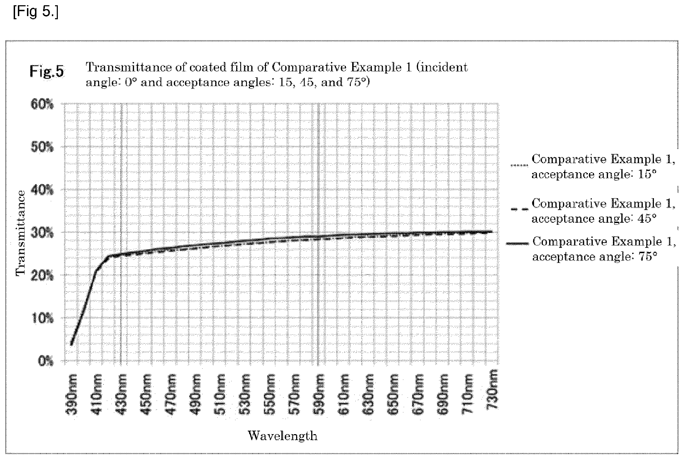

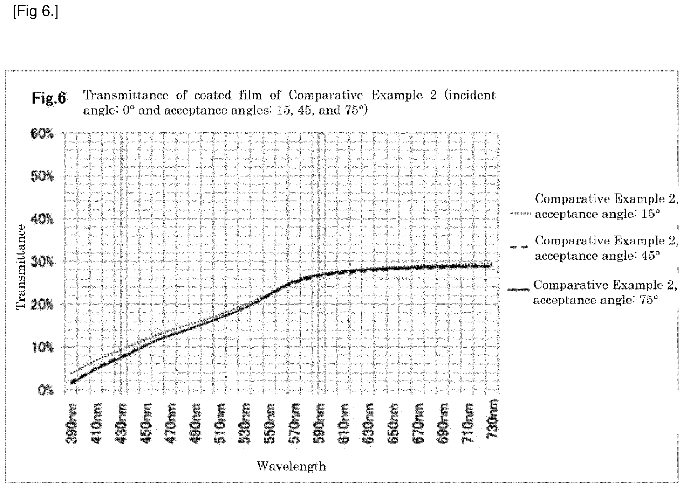

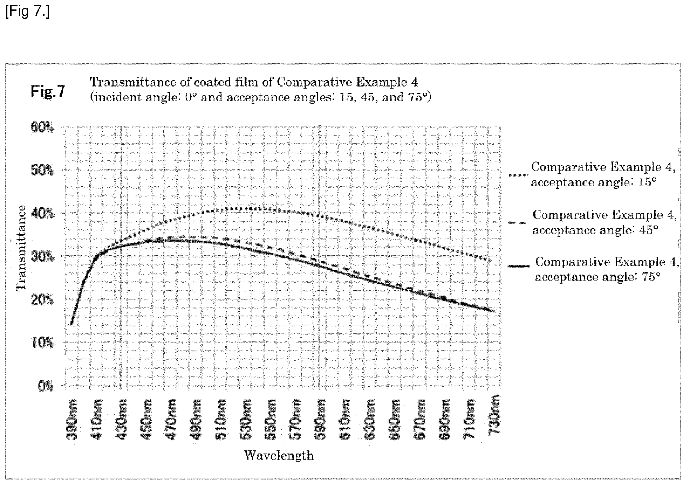

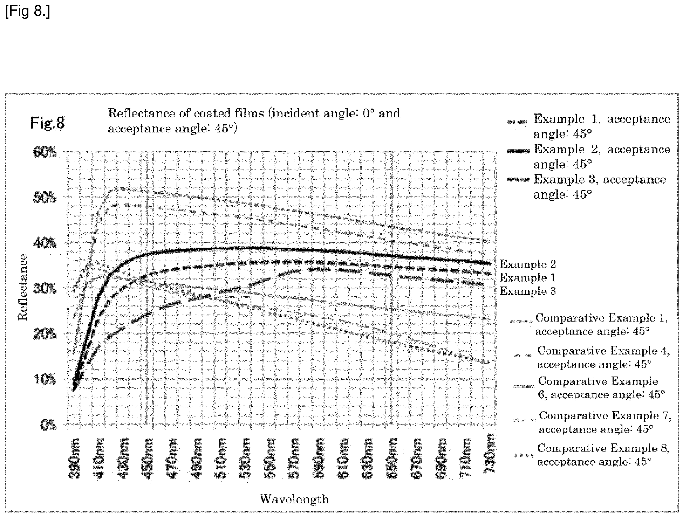

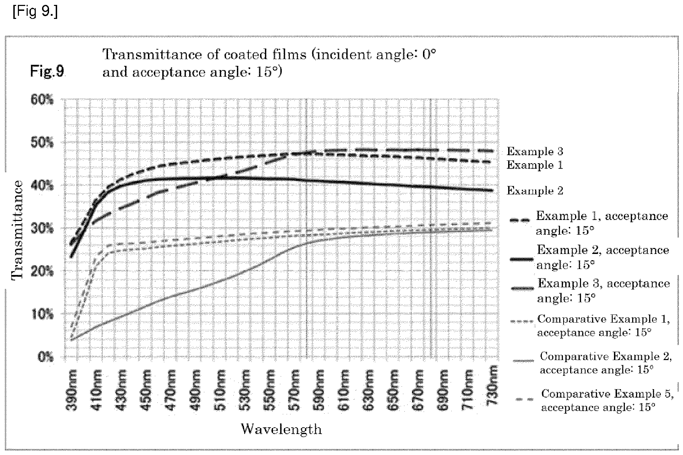

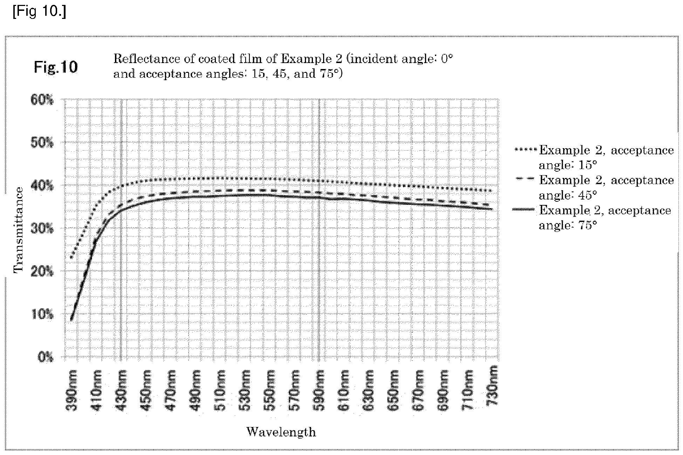

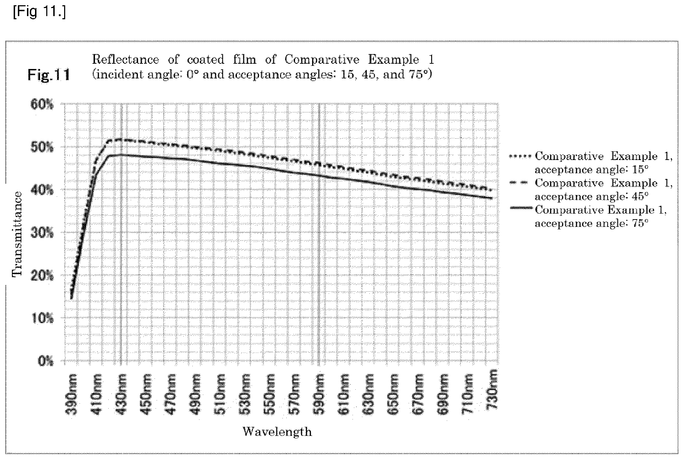

Tables 1 to 3 and Figs. 1 to 11 shows the shapes and morphologies (for example, the sizes and coatings) and optical properties of the particles in the coated titanium oxide powders according to Examples 1 to 3 and Comparative Examples 1 to 8. The denotations *1 to *6 in Table 1 indicate the following commercially available products: *1: MP-701 available from Tayca Corporation; *2: MT-500SA available from Tayca Corporation; *3: MKR-1 available from Sakai Chemical Industry Co., Ltd.; *4: ST-750WD available from Titan Kogyo, Ltd.; *5: ST-643EC available from Titan Kogyo, Ltd.; and *6: ST-710EC available from Titan Kogyo, Ltd.

The mean particle diameter D50 and the aspect ratio of the titanium oxide powder were measured by analysis of transmission electron microscopic images of the titanium oxide powders as described in "Measurement of Mean Particle Diameter D50 and Aspect Ratio by analysis of Transmission Electron Microscopic Image". The mean particle diameter D50 was measured by laser diffraction scattering as described in "Mean Particle Diameter D50, Measured by Laser Diffraction Scattering, of Inventive Coated Titanium Oxide Powder". The metal contents after the surface treatment was measured by an ICP analysis.

The optical properties of the titanium oxide powder were measured as described in "Measurement with Variable Angle Spectrophotometric System".

### Evaluations

Twenty panelists dedicated to evaluation of standard colors for cosmetics participated in the following evaluations and determinations: The skin of each panelist was washed with water and left for 10 minutes. The powder (10%) according to each of Examples and Comparative Examples was dispersed or diluted in talc having a mean particle diameter of about 10 µm. The diluted powder was applied in a specific amount onto the skin with a makeup sponge. The panelist visually rated the powder for evaluation of "natural makeup texture", "shading effect", "pallor", and "yellow dullness". The panelist rated the samples on a scale of 1 to 5 for each evaluation item based on the following "evaluation criteria". The average of the ratings of all the panelists for each evaluation item was also determined on the following "determination criteria":

### Evaluation Criteria

Results Score

| | |
|---|---|
| Very good | 5 |
| Good | 4 |
| Normal | 3 |
| Rather bad | 2 |
| Bad: | 1 |

Determination Criteria
Average of Scores: Determination
4.0 ≤ Average: Good (A)
1.5 ≤ Average < 4.0: Incomplete (B)
Average < 1.5: Bad (C)

The term "Normal" under "Evaluation Criteria" refers to an effect that is neither bad nor good. The term "Average of Scores" under "Determination Criteria" refers to an arithmetic mean value. The terms "Good (A)" and "Bad (C)" in "Determination" under "Determination Criteria" represent "accepted" and "rejected", respectively. In the column "Evaluation: arithmetic mean value (kurtosis) in Table 1, the value in the upper line represents the arithmetic mean value and the parenthesized value in the lower line represents the kurtosis.

The kurtosis of the score. the kurtosis is a statistic representing how much the distribution protrudes from the normal distribution and indicates the sharpness and spread of the distribution. A distribution having a sharp peak at the average and a heavy tail compared with the normal distribution has a positive value. A distribution having a flat peak spreading from the average and a light tail compared to the normal distribution has a negative value. The normal distribution has a kurtosis value of 0.

### Properties of Powder

The titanium oxide powders of Examples 1 to 3 were each coated with a hydrous silica layer, an aluminum hydroxide layer, and an iron oxide layer in sequence. The aluminum hydroxide coating layer of the titanium oxide powder incompletely covered the hydrous silica coating layer; hence, the outermost iron oxide coating layer covered a mixed surface of hydrous silica coating layer and the aluminum hydroxide coating layer. The crystals of the uncoated titanium oxide particles according to Examples 1 to 3 contained 0.2 to 0.7 mass% zinc oxide.

Although Examples 1 to 3 show exemplary processes, it suffices that the finished titanium oxide powder according to the present invention is coated with the hydrous silica coating layer, the aluminum hydroxide coating layer, and then the iron oxide coating layer. The core of the titanium oxide powder may be coated with aluminum hydroxide and then iron oxide. Alternatively, a hydrous-silica-coated titanium oxide powder may be coated with aluminum hydroxide and then iron oxide.

The coated titanium oxide powder according to each of Examples 1 to 3 had a mean particle diameter D50 of 0.2 to 0.4 µm as measured by analysis of transmission electron microscopic images; a mean particle diameter D50 of 0.7 to 1.1 µm as measured by laser diffraction scattering; and an aspect ratio of 1.5 to 1.6. The coated titanium oxide powder according to each of Examples 1 to 3 after surface treatment contained 1 to 5 mass% aluminum oxide, 0.5 to 6 mass% silicon oxide, and 0.5 to 3 mass% iron oxide.

### Evaluations

The coated titanium oxide powders according to Examples 1 to 3 have significantly superior properties in the evaluation items "natural makeup texture" and "shading effect". In contrast, the titanium oxide powders according to Comparative Examples 1 to 8 have unsatisfactory properties in the evaluation items "natural makeup texture" and "shading effect".

Also in the evaluation items "pallor" and "yellow dullness", the coated titanium oxide powders according to Examples 1 to 3 have significantly superior properties. In contrast, the titanium oxide powders according to Comparative Examples 1 to 8 have unsatisfactory properties in the evaluation items "pallor" and "yellow dullness". In particular, the coated titanium oxide powders according to Examples 1 to 2 have significantly superior properties in the evaluation item "yellow dullness" and the coated titanium oxide powder according to Example 3 in "yellow dullness". All the averages of the scores in the evaluation items have high kurtoses without observable distributions.

In particular, the coated titanium oxide powder according to Example 2 among Examples 1 to 3 is the supreme properties in all the evaluation items "natural makeup texture", "shading effect", and "yellow dullness". All the averages of the scores in the evaluation items have high kurtoses without observable distributions.

### Optical Properties

The coated titanium oxide powders according to Examples 1 to 3 each have a higher transmittance at an acceptance angle of 15 degrees than at an acceptance angle of 45 degrees, at both wavelengths of 430 and 580 nm.

In the optical properties of the coated titanium oxide powders according to Examples 1 to 3, the ratio of the transmittance at an acceptance angle of 15 degrees to the transmittance at an acceptance angle of 45 degrees is 1.5 to 2.3, in other words, equal to or higher than 1.5 at a wavelength of 430 nm.

The ratio of the transmittance at an acceptance angle of 15 degrees to the transmittance at an acceptance angle of 45 degrees at a wavelength 430 nm is higher than the ratio of the transmittance at an acceptance angle of 15 degrees to the transmittance at an acceptance angle of 45 degrees at a wavelength of 590 nm. The ratio of the transmittance at 430 nm to the transmittance at 590 nm is 1.2 to 1.5.

In view of the results of the evaluations and the optical properties, a coated titanium oxide powder can achieve a natural makeup texture after the powder is applied to the skin, where the coated titanium oxide powder has a ratio of the transmittance of the coated film at an acceptance angle of 15 degrees to the transmittance of the coated film at an acceptance angle of 45 degrees is 1.3 or more at a wavelength of 430 nm. A coated titanium oxide powder can achieve a preferred natural makeup texture of the skin after being applied to the skin, where the coated titanium oxide powder has a ratio of the transmittance at an acceptance angle of 15 degrees to the transmittance at an acceptance angle of 45 degrees at 430 nm that is equal to or higher than the ratio of the transmittance at an acceptance angle of 15 degrees to the transmittance at an acceptance angle of 45 degrees at 590 nm.

The coated titanium oxide powder having these two optical transmission properties can achieve both a preferred natural makeup texture and shading effect to the skin in makeup.

The coated titanium oxide powders according to Examples 1 to 3 each have a maximum reflectance (the highest reflectance) at an acceptance angle of 45 degrees in a wavelength region of 530 to 590 nm. The reflection spectrum demonstrates that the coated titanium oxide powder has a maximum reflectance at an acceptance angle of 45 degrees in a long wavelength region of 450 nm or more where the wavelength of 450 nm is characteristic to bluishness and the wavelength of 650 nm is characteristic to reddishness.

In each of the coated titanium oxide powders according to Examples 1 to 3, the ratio of the reflectance at a wavelength of 450 nm to the reflectance at a wavelength of 650 nm is 1.1 to 0.7.

The results of the evaluations, the optical properties at 450 nm characteristic to bluishness and at 650 nm characteristic to reddishness conclude that titanium oxide powder provided with a coated film having a maximum reflectance (the highest reflectance) in a long wavelength region of 450 nm or more of a reflection spectrum can more effectively reduce the pallor of the skin after the powder is applied to the skin.

Hence, a coated titanium oxide powder having these optical reflection and transmission properties can effectively achieve not only the natural makeup texture and the shading effect to the skin but also a reduction in pallor of the skin.

The coated titanium oxide powders according to Examples 1 to 3 each have a maximum transmittance of transmitted light at an acceptance angle of 15 degrees in a wavelength region of 510 to 670 nm, preferably 510 to 570 nm. The coated titanium oxide powders according to Examples 1 to 3 each have a transmittance of 40 to 48% at an acceptance angle of 15 degrees and a wavelength of 680 nm characteristic to reddishness.

The results of the evaluations and the optical properties at the wavelength of 680 nm characteristic to reddishness conclude that the titanium oxide powder provided with a coated film having a maximum transmittance (the highest transmittance) in a short wavelength region of 580 nm or less or a transmittance of 30% or more at a wavelength of 680 nm in a transmission spectrum provides no unnatural masking feeling and can more effectively reduce the yellow dullness of the skin after the powder is applied to the skin.

The coated titanium oxide powder having these optical transmission can effectively achieve a natural makeup texture and shading effect to the skin with reduced yellow dullness.

The coated titanium oxide powders according to Examples 1 to 3 exhibits only a small difference of about ±3% in transmittance between at an acceptance angle of 45 degrees and the transmittance at an acceptance angle of 75 degrees.

In conclusion, the coated titanium oxide powder according to the present invention can effectively achieve a natural makeup texture and shading effect to the skin with reduced pallor and yellow dullness in makeup.

The coated titanium oxide powders according to Examples 1 to 3 are particles that are larger and more rounded than conventional titanium oxide powder particles and thus can well spread over the skin. The coated titanium oxide powder according to the present invention has such a shape and morphology that can effectively achieve a natural makeup providing a fresh feeling of the bare skin to everyone even if being slapped on the skin.

A dispersion of the coated titanium oxide powder (30 mass%) diluted with talc was applied to a transparent tape with a brush. The coated tape was placed in a glass container for visual observation of the transmitted light using halogen white light. As a result, it was visibly confirmed that the coated titanium oxide powder according to the present invention transmitted not the yellow dull light but the white light and provide no unnatural hiding feeling despite the thick coated film.

Color unevenness observing films for blue, green, yellow, and red colors were placed onto a pseudo skin sheet. The coated titanium oxide powder was applied to the films with a brush. The films were visually observed. The results of the visual observation of the films demonstrate that the films exhibit color tones fitting the pseudo skin sheet and an appropriate masking effect.

In this way, the coated titanium oxide powder according to the present invention can transmit the direct light, in particular, not the yellow dull light but the white light from the interior of the skin at a substantially right angle and achieve a natural shading covering effect without a hiding feeling, where the visible reddish light is transmitted from the sides of the face, not the directly transmitting pallid light.

In makeup, a visual check is also important. From the viewpoint of the visual check, the coated titanium oxide powder according to the present invention can maintain an appropriate covering effect (the so-called masking effect) that can be partially adjusted by a user in makeup.

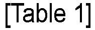

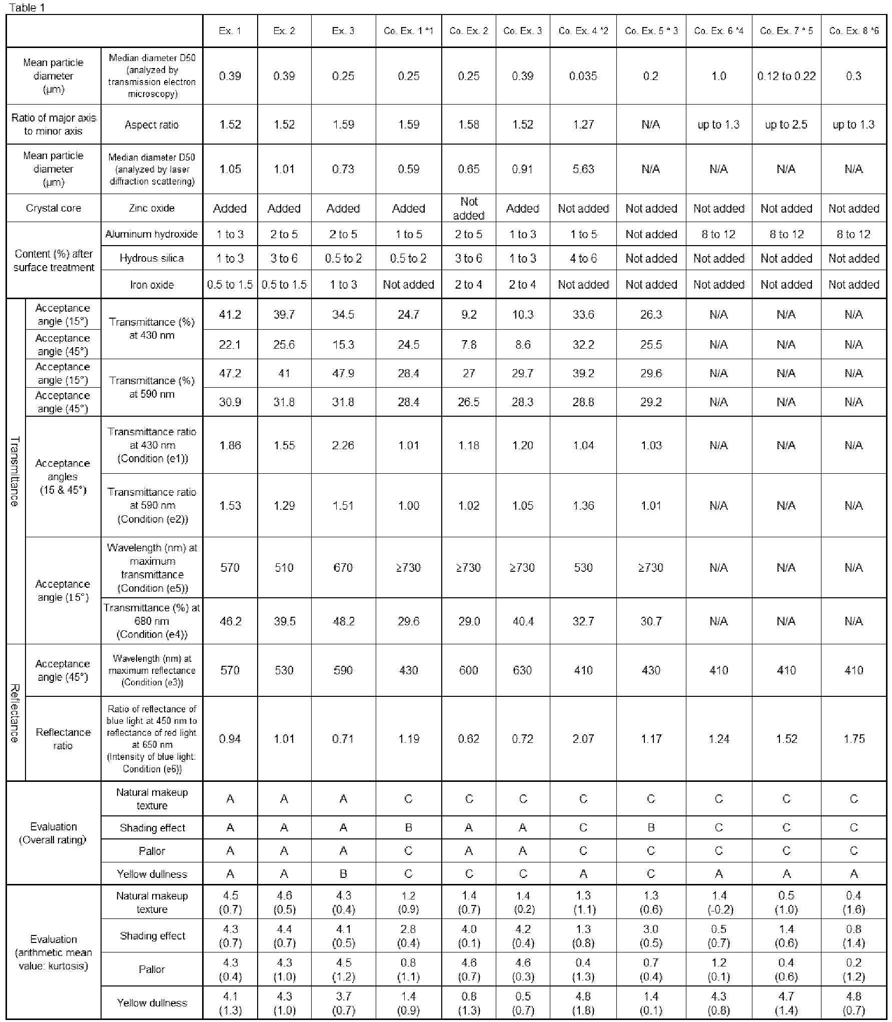

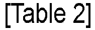

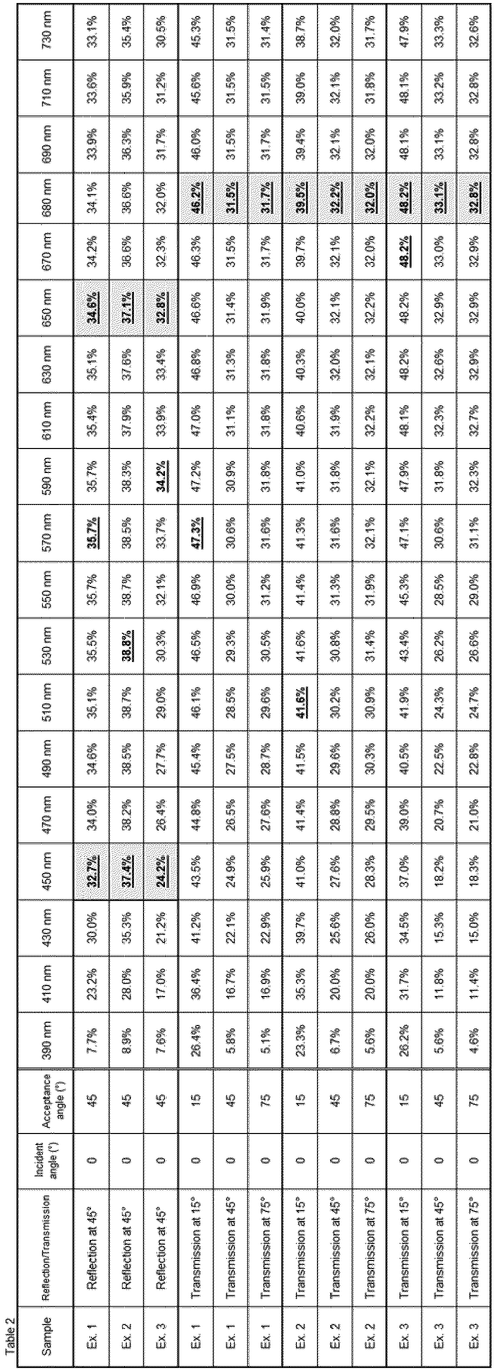

The present invention will now be described in more detail by way of Product Examples and Comparative Product Examples prepared with the novel coated titanium oxide powders according to Examples 1 to 3 or processed powders of the novel coated titanium oxide powders as described below. Product Examples and Comparative Product Examples should not be construed to limit the present invention. Each product was evaluated on the same criteria as those in the evaluations of the powders, where the product was applied in a specific amount onto the skin like a common cosmetic.

### Product Examples 1 to 3 (solidified powder cosmetic compositions: powdered foundation compositions)

Product Examples 1 to 3 (solidified powder cosmetic compositions or powdered foundation compositions) will now be described that are prepared with the novel titanium oxide powders according to Examples 1 to 3, where the titanium oxide powders are coated with 0.2% dimethicone. The product prepared with Example 1 is referred to as Product Example 1, the product with Example 2 as Product Example 2, and the product with Example 3 as Product Example 3. These Product Examples should not be construed to limit the present invention.

### Component

1. Inventive titanium oxide according to each of Examples 1 to 3 (treated with 0.2% dimethicone) 10.0%
2. Silicone-treated titanium oxide (mean particle diameter: 0.035 µm) *1 5.0%
3. Dimethiconol and aminopropyl triethoxysilane-treated talc *2 20.0%
4. Magnesium/potassium/silicon fluoride/hydroxide/oxide *3 5.0%
5. Mica the balance
6. Boron nitride (mean particle diameter: 15.5 µm) 10.0%
7. Dimethicone-treated zinc oxide (mean particle diameter: 35 nm) 2.0%
8. Yellow iron oxide 1.5%
9. Red iron oxide 0.5%
10. Black iron oxide 0.2%
11. Crosspolymer of HDI, PPG, and polycaprolactone *4 8.0%
12. Crosspolymer of diphenyl dimethicone, vinyl diphenyl dimethicone, and silsesquioxane *5 5.0%
13. Nylon powder 5.0%
14. 2-ethylhexyl methoxycinnamate 5.0%
15. Dimethyl polysiloxane 3.0%
16. Squalene 3.0%
17. PEG-11 methyl ether dimethicone *6 0.5%
18. Glycerin mono-2-ethylhexyl ether 0.2%
19. Fragrance as required
   *1: SMT-500SAM available from Tayca Corporation
   *2: Talc JA-13R available from Asada Milling Co., Ltd. (treatment by 2.8%)
   *3: Micro Mica MK-200K available from Katakura & Co-op Agri Corporation)
   *4: PLASTIC POWDER CS-400 available from Negami Chemical Industrial Co., Ltd.
   *5: KSP-300 available from Shin-Etsu Chemical Co., Ltd.
   *6: KF-6018 available from Shin-Etsu Chemical Co., Ltd.

### Process

(1): Components 1 to 13 were homogenized with a Henschel mixer.
(2): Components 14 to 19 were thoroughly mixed.
(3): Mixture (2) was added to components (1), homogenized, and then pulverized.
(4): Mixture (3) was mixed with appropriate amounts of purified water and alcohol to form a slurry mixture.
(5): Slurry mixture (4) was placed and compacted in a metal pan. Excess water was removed. The product was then dried to remove the solvent, resulting in a solidified powder foundation composition.

### Evaluations

The powdered foundation compositions according to Product Examples 1 to 3 prepared with the novel titanium oxide powders of Examples 1 to 3 were rated for evaluation items "natural makeup texture", "shading effect", "pallor", and "yellow dullness". The powdered foundation compositions had high scores in all the evaluation items.

### Product Example 4 (solidified powder cosmetic composition: powdered foundation composition 2)

### Component

1. Inventive titanium oxide according to Example 2 (treated with 0.2% hydrogenated lecithin) 8.0%
2. Titanium oxide (mean particle diameter: 1.0 µm) 5.0%
3. Silicone-treated talc (mean particle diameter: 10 µm) 30.0%
4. Synthetic ferrous golden mica 15.0%
5. Platy barium sulfate 5.0%
6. Triethoxycaprylylsilane-treated mica the balance
7. Red iron oxide 0.2%
8. Yellow iron oxide 1.5%
9. Black iron oxide 0.2%
10. Crosslinked poly(methyl methacrylate) *7 5.0%
11. Silica *8 1.0%
12. N-lauroyl-L-lysine *9 8.0%
13. Methylphenyl polysiloxane 3.0%
14. 2-ethylhexyl methoxycinnamate 3.0%
15. Liquid paraffin 3.0%
16. Beauty component 1.0%
17. Fragrance 0.1%
18. Phenoxyethanol 0.1%
   *7: Techpolymer MBP-8 available from Sekisui Kasei Co., Ltd.
   *8: Cosme Silica CQ4 available from Fuji Silysia Chemical LTD.
   *9: AMIHOPE LL available from Ajinomoto Co., Inc.

### Process

(1): Components 1 to 12 were homogenized and mixed with a Henschel mixer.
(2): Components 13 to 18 were thoroughly mixed.
(3): Mixture (2) was added to mixture (1) and homogenized.
(4): Mixture (3) was pulverized.
(5): Product (4) was placed and compacted in a metal pan to produce a solidified powder foundation composition.

### Evaluations

The solidified powder foundation composition according to Product Example 4 was rated for evaluation items "natural makeup texture", "shading effect", "pallor", and "yellow dullness". The solidified powder foundation had high scores in all the evaluation items.

### Product Example 5 (oily solid cosmetic composition: UV protective foundation composition)

### Component

1. Talc 15.0 mass%
2. Fired sericite 10.0 mass%
3. Inventive titanium oxide according to Example 3 (treated with 0.2% dimethicone) 15.0 mass%
4. Silicone-treated microparticulate titanium oxide (coated with 40% sericite) 10.0 mass%
5. Silicone-treated red iron oxide 1.0 mass%
6. Silicone-treated yellow iron oxide 3.0 mass%
7. Silicone-treated black iron oxide 0.2 mass%
8. Polyethylene wax 7.0 mass%
9. Microcrystalline wax 6.0 mass%
10. Glyceryl tri(2-ethylhexanoate) the balance
11. Stearoxy dimethicone 3.0 mass%
12. Liquid paraffin 20.0 mass%
13. Copolymer of polyoxyethylene methyl siloxane, polyoxypropylene oleyl methyl siloxane, and dimethylsiloxane *10 2.0 mass%
14. Antiseptic agent as required
15. Fragrance 0.1 mass%
   *10: KF-6026 available from Shin-Etsu Chemical Co., Ltd.

### Process

(1): Components 8 to 14 were mixed at 90°C.
(2): Component 1 to 7 were added to mixture (1) and homogenized with a roll mill.
(3): Component 15 was added to the mixture (2) at 80°C and then placed in a metal pan to produce an oily solid UV protective foundation composition.

### Evaluations

The oily solid UV protective foundation composition according to Product Example 5 was rated for evaluation items "natural makeup texture", "shading effect", "pallor", and "yellow dullness. The oily solid UV protective foundation composition had high scores in all the evaluation items.

### Product Example 6 (oily solidified powder cosmetic composition: foundation composition)

### Component

1. Inventive titanium oxide according to Example 1 (treated with 0.2% dimethicone) 15.0%
2. Zinc oxide (mean particle diameter: 3 µm) *11 5.0%
3. Talc 10.0%
4. Mica the balance
5. Red iron oxide 0.2%
6. Yellow iron oxide 1.5%
7. Black iron oxide 0.2%
8. Crosspolymer of butyl acrylate and glycol dimethacrylate, crosspolymer of methyl methacrylate and glycol dimethacrylate crosspolymer, and silica *12 15.0%
9. Silica *13 1.0%
10. Crosspolymer of dimethicone and vinyl dimethicone *14 8.0%
11. Methylphenyl polysiloxane 15.0%
12. Decamethylcyclopentasiloxane 10.0%
13. Liquid paraffin 5.0%
14. Polyethylene wax 2.0%
15. 1,2-Butanediol 0.1%
16. Fragrance 0.1%
   *11: XZ-3000F available from Sakai Chemical Industry Co., Ltd.
   *12: Matsumoto Microsphere S-100 available from Matsumoto Yushi-Seiyaku Co., Ltd.
   *13: Godd Ball E2-824C available from SUZUKIYUSHI INDUSTRIAL CORPORATION
   *14: KSG-16 available from Shin-Etsu Chemical Co., Ltd.

### Process

(1): Components 10 to 11 were homogenized and swollen.
(2): Components 13 and 14 were thoroughly mixed at 110°C.
(3): Mixture (1) was added to components 1 to 9 and rolled. Mixture (2) and components 12, 15, and 16 were added to the rolled product and kneaded.
(4): Kneaded product (3) or an oily foundation composition was placed in an air-tight container.

### Evaluations

The oily foundation composition according to Product Example 6 was rated for evaluation items "natural makeup texture", "shading effect", "pallor", "yellow dullness". The oily foundation composition had high scores in all the evaluation items. Even if component 12 "decamethylcyclopentasiloxane" was replaced with "dimethyl polysiloxane (2 cs)", the foundation composition had high scores in the evaluation items "natural makeup texture", "shading effect", "pallor" and "yellow dullness" as in Product Example 6.

### Product Example 7 (oily powder cosmetic composition: blusher)

### Formulation

1. Microcrystalline wax 2.0%
2. Dimethicone (2 cs) 10.0%
3. Bis(2-ethylhexyl) succinate 4.0%
4. Decamethylcyclopentasiloxane 5.0%
5. Crosspolymer of dimethicone and vinyl dimethicone *14 20.0%
6. Methylphenyl polysiloxane 10.0%
7. Trimethylsiloxysilicate 5.0%
8. Inventive titanium oxide according to Example 2 (treated with 0.2% aluminum distearate) 2.0%
9. Red 226 (Color Index number Vat Red 1) 0.5%
10. Yellow 4 (Color Index number Acid Yellow 23) 1.0%
11. Blue 1 (Color Index number Food Blue 2) 0.1%
12. Red iron oxide 0.5%
13. Yellow iron oxide 0.3%
14. Silica anhydride (mean particle diameter: 30 µm) 3.0%
15. Polyethylene powder *15 5.0%
16. Block copolymer of crosslinked silicone and network silicone *16 5.0%
17. Chlorphenesin 0.2%
18. Silicon-treated sericite the balance
19. Synthetic ferrous golden mica (mean particle diameter: 20 µm) 5.0%
   *15: MIPELON XM-220 available from Mitsui Chemicals, Inc.
   *16: KSP-102 available from Shin-Etsu Chemical Co., Ltd.

### Process

(1): Components 1 to 3 were thoroughly mixed at 90°C.
(2): Components 4 to 7 were added to and mixed with mixture (1).
(3): Components 8 to 19 were added to mixture (2) and homogenized.
(4): Mixture (3) was kneaded under reduced pressure.
(5): Kneaded product (4) was placed in a container and shaped under pressure to produce a blusher.

### Evaluations

The blusher according to Product Example 7 was rated for evaluation items "natural makeup texture", "shading effect", "pallor", and "yellow dullness". The oily powder cosmetic composition had high scores in all the evaluation items. Even if component 4 "decamethylcyclopentasiloxane" was replaced with "dimethyl polysiloxane (1.5 cs)", the oily powder cosmetic composition had high scores in the evaluation items "natural makeup texture", "shading effect", "pallor", and "yellow dullness" as in Product Example 7.

### Product Example 8 (oil-in-water cosmetic composition: liquid foundation composition)

### Component

1. Inventive titanium oxide according to Example 1 (treated with 0.3% isopropyl titanium triisostearate) 6.0%
2. Inventive titanium oxide according to Example 3 (treated with 0.5% hydrogenated lecithin) 2.0%
3. Red iron oxide (treated with 0.3% hydrogenated lecithin) 0.2%
4. Yellow iron oxide (treated with 0.5% hydrogenated lecithin) 1.4%
5. Black iron oxide (treated with 0.3% hydrogenated lecithin) 0.1%
6. Lecithin-treated oxidation talc (mean particle diameter: 5 to 10 µm) 1.0%
7. Silica (mean particle diameter: 1 to 5 µm) 0.5%
8. PEG-10 hydrogenated castor oil 0.5%
9. Polysorbate 80 0.5%
10. Triceteareth-4 phosphate 0.1%
11. 1,3-Butylene glycol 7.0%
12. Triethanolamine 1.5%
13. Purified water the balance
14. Stearic acid 1.0%
15. Glyceryl monostearate 0.5%
16. Cetearyl alcohol 0.5%
17. Behenyl alcohol 0.5%
18. Ethylhexyl methoxycinnamate 4.0%
19. Diethylamino hydroxybenzoyl hexyl benzoate 1.0%
20. Liquid paraffin 5.0%
21. Triethylhexanoin 7.0%
22. Dimethyl polysiloxane (2 cs) 3.0%
23. Purified water 15.0%
24. Glycerin 3.0%
24. Carboxyvinyl polymer 0.2%
25. Crosspolymer of acrylates and alkyl acrylate (C10-30) 0.1%
26. Ethanol 5.0%
27. Methylene bis-benzotriazolyl tetramethylbutylphenol 3.0%
28. Polyglyceryl-10 laurate 0.5%

### Process

(1): Components 1 to 11 were homogenized with a roll mill.
(2): Components 12 to 13 were thoroughly mixed.
(3): Mixture (1) was added to and thoroughly mixed with mixture (2).
(4): Components 14 to 22 were mixed at 80°C.
(5): Mixture (4) was added to and emulsified in mixture (3) at 80°C.
(6): Emulsion (5) was cooled. Components 23 to 28 were added to the emulsion to produce an oil-in-water foundation composition.

### Evaluations

The oil-in-water foundation composition according to Product Example 8 was rated for evaluation items "natural makeup texture", "shading effect", "pallor", and "yellow dullness". The oil-in-water foundation composition had high scores in all the evaluation items.

### Product Example 9 (water-in-oil cosmetic composition: liquid foundation composition)

### Component

1. Inventive titanium oxide according to Example 2 (treated with 0.3% disodium N-stearoyl-L-glutamate 4.0%
2. Silicone-treated titanium oxide (mean particle diameter: 0.7 µm) 2.0%
3. Titanium oxide (treated with 3% dimethyl polysiloxane: mean particle diameter: 35 nm) 6.0%
4. Silicone-treated zinc oxide (hexagonal plates; mean particle diameter: 0.3 µm) 5.0%
5. Red iron oxide (treated with 2% octyltriethoxysilane) 0.2%
6. Yellow iron oxide (treated with 2% octyltriethoxysilane) 1.1%
7. Black iron oxide (treated with 2% octyltriethoxysilane) 0.1%
8. Talc (treated with 2% dimethyl polysiloxane) 1.0%
9. PEG-9 polydimethylsiloxyethyl dimethicone 0.3%
10. Lauryl PEG-9 polydimethylsiloxyethyl dimethicone 0.5%
11. Lauryl polyglyceryl-3 polydimethylsiloxyethyl dimethicone 1.0%
12. Ethylhexyl methoxycinnamate 4.0%
13. Triethylhexanoin 1.0%
14. Bis(2-ethylhexyl) succinate 1.0%
15. Diisostearyl malate 0.5%
16. Shea butter 0.5%
17. Decamethylcyclopentasiloxane 5.0%
18. Dimethyl polysiloxane (3.5 cs) 1.0%
19. Methyl trimethicone 8.0%
20. Diphenylsiloxy phenyl trimethicone 5.0%
21. Phenoxyethanol 0.3%
22. Purified water the balance
23. Ethanol 6.0%
24. Tripropylene glycol 5.0%
25. Dipropylene glycol 1.0%
26. PEG-240/decyltetradeceth-20 copolymer 0.3%
27. Tremella fuciformis polysaccharide 0.1%

### Process

(1): Components 1 to 11 were homogenized.
(2): Components 12 to 21 were thoroughly mixed.
(3): Mixture (1) was added to and thoroughly mixed with mixture (2).
(4): Components 22 to 27 were mixed.
(5): Mixture (4) was added to and emulsified in mixture (3) to produce a water-in-oil liquid foundation composition.

### Evaluations

The water-in-oil liquid foundation composition according to Product Example 9 was rated for evaluation items "natural makeup texture", "shading effect", "pallor", and "yellow dullness". The water-in-oil liquid foundation composition had high scores in all the evaluation items. Even if component 17 "decamethylcyclopentasiloxane" in Product Example 9 was replaced with "dimethyl polysiloxane (2 cs)", the water-in-oil liquid foundation composition had high scores in the evaluation items "natural makeup texture", "shading effect", "pallor", and "yellow dullness" as in Product Example 9.

### Product Example 10 (water-in-oil cosmetic composition: base or sunscreen composition)

### Component

1. Inventive titanium oxide according to Example 3 (treated with 0.5% dimethicone) 3.0%
2. Titanium oxide (treated with 5% dimethicone; mean particle diameter: 35 nm) 2.0%
3. Zinc oxide (treated with 5% dimethicone; mean particle diameter: 25 nm) 6.0%
4. Red iron oxide (treated with 2% octyltriethoxysilane) 0.1%
5. Yellow iron oxide (treated with 2% octyltriethoxysilane) 0.3%
6. Black iron oxide (treated with 2% octyltriethoxysilane) 0.01%
7. Talc (treated with 2% dimethyl polysiloxane) 1.0%
8. PEG-9 polydimethylsiloxyethyl dimethicone 2.0%
9. Lauryl PEG-9 polydimethylsiloxyethyl dimethicone 1.0%
10. Cetyl PEG/PPG-10/1 dimethicone 1.0%
11. Ethylhexyl methoxycinnamate 7.0%
12. Diethylamino hydroxybenzoyl hexyl benzoate 1.0%
13. Bis-ethylhexyloxyphenol methoxyphenyl triazine 1.0%
14. Isotridecyl isononanoate 3.0%
15. Meadowfoam seed oil 1.0%
16. Methyl trimethicone 5.0%
17. Isododecane 10.0%
18. Decamethylcyclopentasiloxane 5.0%
19. Disteardimonium hectorite 1.0%
20. Stearalkonium hectorite 1.0%
21. Polymethylsilsesquioxane 1.0%
22. Polyethylene 2.0%
23. Polypropylene 0.1%
24. Crystalline cellulose 2.0%
25. Silica 1.0%
26. Purified water the balance
27. Ethanol 4.0%
28. 1,3-Butylene glycol 0.5%
29. Glycerin 3.0%
30. Sodium chloride 0.2%
31. Methylene bis-benzotriazolyl tetramethylbutylphenol 1.5%
32. Polyglyceryl-10 laurate 0.3%

### Process

(1): Components 1 and 8 and components 18 to 20 were homogenized with a roll mill.
(2): Components 9 to 13 were thoroughly mixed.
(3): Mixture (1) and components 14 to 17 and 21 to 25 were added to mixture (2) and then thoroughly mixed.
(4): Components 26 to 30 were mixed.
(5): Mixture (4) was added to and emulsified in mixture (3).
(6): Emulsion (5) was cooled. Components 31 to 32 were added to the cooled emulsion to produce a water-in-oil-based sunscreen composition.

### Evaluations

The water-in-oil-based sunscreen composition according to Product Example 10 was rated for evaluation items "natural makeup texture", "shading effect", "pallor", and "yellow dullness". The water-in-oil cosmetic composition had high scores in all the evaluation items. Even if component 18 "decamethylcyclopentasiloxane" in Product Example 10 was replaced with "dimethyl polysiloxane (1.5 cs)", the water-in-oil-based sunscreen composition had high scores in the evaluation items "natural makeup texture", "shading effect", "pallor", and "yellow dullness" as in Product Example 10. The water-in-oil-based sunscreen composition according to Product Example 10 that was injected with an appropriate amount of liquefied petroleum gas into an aerosol spray also had similar high scores.

### Product Example 11 (oily solid cosmetic composition: concealer stick) Component

1. Paraffin wax 5.0%
2. Polyethylene wax 5.0%
3. Candelilla wax 2.0%
4. Propylene glycol dicaprate the balance
5. Methylphenyl polysiloxane 25.0%
6. Petrolatum 5.0%
7. 2-ethylhexyl paramethoxycinnamate 5.0%
8. Inventive titanium oxide according to Example 3 (treated with 0.5% dextrin palmitate) *17 20.0%
9. Yellow iron oxide (treated with dextrin palmitate) *17 2.0%
10. Red iron oxide (treated with dextrin palmitate) *17 0.5%
11. Black iron oxide (treated with dextrin palmitate) *17 0.2%
12. Mica 7.0%
13. Polyhydroxystearic acid 1.0%
   *17: Dextrin palmitate that is available from Chiba Flour Milling Co., Ltd. under the product name of Rheopearl KL.

### Process

(1): Components 1 to 7 were mixed at 100°C.
(2): Components 8 to 13 were thoroughly mixed with mixture (1) at 90°C.
(3): Mixture (2) was processed with a triple-roll mill.
(4): Mixture (3) was defoamed at 85°C, placed in a capsule, and then cooled at 4°C to produce a concealer stick.

### Evaluations

The concealer stick according to Product Example 11 was rated for evaluation items "natural makeup texture", "shading effect", "pallor", and "yellow dullness". The concealer stick had high scores in all the evaluation items.

### Product Example 12 (solidified powder cosmetic composition: eye shadow composition)

### Component

1. Synthetic ferrous golden mica 10.0%
2. Talc the balance
3. Titanium-oxide coated mica 30.0%
4. Boron nitride 5.0%
5. Laminated powder of a poly(ethylene terephthalate), aluminum, and epoxy 5.0%
6. Inventive titanium oxide according to Example 2 (treated with 0.5% zinc laurate) 2.0%
7. Pigment Red 57 0.5%
8. Nylon 12 1.0%
9. 1,2-butanediol as required
10. Liquid paraffin 3.0%
11. Dimethyl polysiloxane (6 cs) 5.0%
12. Glyceryl 2-ethylhexanoate 3.0%
13. Fragrance as required

### Process

(1): Components 1 to 9 were homogenized with a Henschel mixer (available from MITSUI MIIKE MACHINERY CO., LTD.) at 75°C.
(2): Components 10 to 12 were thoroughly mixed.
(3): While mixture (1) was stirred with the Henschel mixer, mixture (2) and component 13 were added and homogenized.
(4): Mixture (3) was pulverized with a pulverizer.
(5): Product (4) was placed and compacted in a metal pan to produce a solidified powder eye shadow composition.

### Evaluations

The eye shadow composition according to Product Example 12 was rated for evaluation items "natural makeup texture", "shading effect", "pallor", and "yellow dullness". The eye shadow composition had high scores in all the evaluation items.

### Product Example 13 (solidified powder cosmetic composition: face color composition)

### Component

1. Amino-modified silicone-treated mica 20.0%
2. Talc treated with zinc laurate the balance
3. Inventive titanium oxide according to Example 2 (treated with 0.1% ceramide II) 10.0%
4. Ultramarine 0.5%
5. Red 226 (Color Index number Vat Red 1) 0.2%
6. Spherical cellulose powder (mean particle diameter: 10 µm) 1.0%
7. Antiseptic agent as required
8. Liquid paraffin 2.0%
9. Dimethyl polysiloxane (6 cs) 3.0%
10. Glyceryl 2-ethylhexanoate 3.0%
11. Ascorbyl magnesium phosphate 0.3%

### Process

(1): Components 1 to 7 were homogenized with a Henschel mixer (available from MITSUI MIIKE MACHINERY CO., LTD.) at 75°C.
(2): Components 8 to 10 were thoroughly mixed at 65°C.
(3): While mixture (1) was stirred with the Henschel mixer, mixture (2) and component 11 were added and homogenized.
(4): Mixture (3) was pulverized with a pulverizer.
(5): Product (4) was placed and compacted in a metal pan to produce a solidified powder face color composition.

### Evaluations

The face color composition according to Product Example 13 was rated for evaluation items "natural makeup texture", "shading effect", "pallor", and "yellow dullness". The face color composition had high scores in all the evaluation items.

### Product Example 14 (powdered cosmetic composition: loose face powder or oshiroi)

### Component

1. Crosspolymer of vinyl dimethicone and methicone silsesquioxane 20.0%
2. Crosspolymer of spherical methyl methacrylate (mean particle diameter: 15.0 µm) 10.0%
3. N-lauroyl-L-lysine 10.0%
4. Synthetic ferrous golden mica 5.0%
5. Silica 2.0%
6. Boron nitride 2.0%
7. Chlorphenesin 0.2%
8. Inventive titanium oxide according to Example 1 (treated with 0.05% polyglutamic acid) 1.0%
9. Talc (mean particle diameter: 20 µm) the balance
10. Yellow iron oxide 0.1%
11. Red iron oxide 0.5%
12. Black iron oxide 0.1%
13. Phenoxyethanol 0.2%
14. Glyceryl tri(2-ethylhexanoate) 0.2%
15. Ethanol 0.5%
16. Fragrance 0.5%

### Process

(1): Components 1 to 12 were thoroughly mixed with a supermixer.
(2): Components 13 to 16 were thoroughly mixed.
(3): Mixture (2) was added to and thoroughly mixed with mixture (1).
(4): Mixture (3) was pulverized to produce a loose face powder or oshiroi that was then placed in a container.

### Evaluations

The loose face powder according to Product Example 14 was rated for evaluation items "natural makeup texture", "shading effect", "pallor", and "yellow dullness". The oshiroi powder had high scores in all the evaluation items.

### Product Example 15 (oily cosmetic composition: lip rouge stick)

### Component

1. Polyethylene wax 10.0%
2. Carnauba wax 5.0%
3. Paraffin wax 2.0%
4. Cetyl 2-ethylhexanoate the balance
5. Liquid paraffin 10.0%
6. Polyglyceryl-2 triisostearate 10.0%
7. Octyldodecanol 5.0%
8. Red 202 (Color Index number Pigment Red 57) 0.5%
9. Yellow 4 (Color Index number Acid Yellow 23) 2.0%
10. Carmine-coated mica titanium 3.0%
11. Inventive titanium oxide according to Example 1 (treated with 0.4% lecithin) 0.5%
12. Black iron oxide 0.1%
13. α-tocopherol 0.5%
14. Fragrance as required

### Process

(1): Components 1 to 7 were mixed at 100°C.
(2): Components 8 to 14 were added to and thoroughly mixed with mixture (1).
(3): Mixture (2) was poured in a container and cooled to produce a lip rouge stick.

### Evaluations

The lip rouge stick according to Product Example 15 was rated for evaluation items "natural makeup texture", "shading effect", "pallor", and "yellow dullness". The lip rouge stick had high scores in all the evaluation items.

### Product Example 16 (oil-in-water cosmetic composition: mascara composition) Component

1. Stearic acid 2.0%
2. Bees wax 10.0%
3. Cetostearyl alcohol 1.0%
4. Polyoxyethlyene sorbitan monooleate (20EO) 1.5%
5. Sorbitan sesquioleate 0.5%
6. Dimethylpolysiloxane 5.0%
7. Inventive titanium oxide according to Example 1 (treated with 0.3% N-lauroyl-L-lysine) 0.2%
8. Red iron oxide 3.0%
9. Purified water the balance
10. 1,3-Butylene glycol 10.0%
11. Triethanolamine 1.5%
12. Emulsion of alkyl acrylate copolymer *18 30.0%
13. Antiseptic agent as required
14. Fragrance as required
   *18: lodozol 32A707 (in a solid content of 45%) available from Japan NSC Co., Ltd.

### Process

(1): Components 1 to 3 were thoroughly mixed at 80°C.
(2): Components 4 to 8 were processed with a roll mill.
(3): Components 9 to 14 were thoroughly mixed at 80°C.
(4): Mixture (3) was added to and emulsified with the mixtures (1) and (2).
(5): Emulsion (4) was cooled to produce an oil-in-water mascara composition.

### Evaluations

The oil-in-water mascara composition according to Product Example 16 was rated for evaluation items "natural makeup texture", "shading effect", "pallor", and "yellow dullness". The oil-in-water mascara composition had high scores in all the evaluation items.

### Product Example 17 (oily cosmetic composition: non-aqueous mascara composition)

### Component

1. Pentaerythritol rosinate 10.0%
2. Candelilla resin 3.0%
3. Bees wax 2.0%
4. Ceresin wax 2.0%
5. Dextrin myristate 2.0%
6. Trimethylsiloxysilicate 3.0%
7. Dimethyldistearyl ammonium hectorite 5.0%
8. Propylene carbonate 1.0%
9. Light liquid isoparaffin the balance
10. Dimethylpolysiloxane 3.0%
11. Inventive titanium oxide according to Example 2 (treated with 1.0% N-lauroyl-L-lysine) 0.3%
12. Silicone-treated ultramarine 3.0%
13. Talc 5.0%
14. Silica 3.0%

### Process

(1): Components 1 to 5 were mixed at 110°C.
(2): Components 6 to 10 were added to and mixed with mixture (1).
(3): Components 11 to 14 were added to mixture (2).
(4): Mixture (3) was processed with a triple-roll mill to produce a non-aqueous mascara composition.

### Evaluations

The non-aqueous mascara composition according to Product Example 17 was rated for evaluation items "natural makeup texture", "shading effect", "pallor", and "yellow dullness". The non-aqueous mascara composition had high scores in all the evaluation items.

### Product Example 18 (oily cosmetic composition: eye liner composition) Component

1. Ceresin wax 11.0%
2. Polyisobutylene 16.0%
3. Polyethylene wax 8.0%
4. Light liquid isoparaffin the balance
5. Dimethylpolysiloxane (1.5cs) 3.0%
6. Iron blue 10.0%
7. Inventive titanium oxide according to Example 2 (treated with 0.3% silicon (product name: KP-562P)) 1.0%
8. Talc 5.0%
9. Antiseptic agent as required
10. Fragrance as required

### Process

(1): Components 1 to 5 were thoroughly mixed at 100°C.
(2): Components 6 to 10 were thoroughly mixed at 80°C.
(3): Mixture (2) was added to and thoroughly mixed with mixture (1).
(4): Mixture (3) is processed with a roll mill to produce an oily eye liner composition.

### Evaluations

The oily eye liner composition according to Product Example 18 was rated for evaluation items "natural makeup texture", "shading effect", "pallor", and "yellow dullness". The oily eye liner composition had high scores in all the evaluation items.

## Claims

1. A coated titanium oxide powder comprising a coating layer of a silicon oxide or hydroxide, a coating layer of an aluminum oxide or hydroxide, and a coating layer of an iron oxide or hydroxide, wherein
the coated titanium oxide powder has a mean particle diameter D50 of 0.20 to 0.45 µm measured by analysis of a transmission electron microscopic image,
the coated titanium oxide powder has a mean particle diameter D50 of 0.6 to 1.2 µm measured by laser diffraction scattering, and
the coated titanium oxide powder in a form of a coated film has a ratio of the transmittance of transmitted light at an acceptance angle of 15 degrees to the transmittance of transmitted light at an acceptance angle of 45 degrees of 1.3 or more at a wavelength of 430 nm, and the ratio of the transmittance of transmitted light at an acceptance angle of 15 degrees to the transmittance of transmitted light at an acceptance angle of 45 degrees at a wavelength of 430 nm is equal to or higher than the ratio of the transmittance of transmitted light at an acceptance angle of 15 degrees to the transmittance of transmitted light at an acceptance angle of 45 degrees at a wavelength of 590 nm, where
the coated film is prepared by dispersing the coated titanium oxide comprising a pure titanium oxide content in an amount of net 10 mass% and a copolymer of acrylate and dimethicone in an amount of 20 mass% in a volatile solvent, applying the dispersion onto a glass plate into a thickness of 25 µm, and drying the applied layer, and the transmitted light is generated by irradiation of the coated film with light from a CIE standard light source D65 of a variable angle spectrophotometric system at an incident angle of 0 degrees.

2. The coated titanium oxide powder according to claim 1, wherein the coated film has a maximum reflectance in a long wavelength region of 450 nm or more in a reflection spectrum at an acceptance angle of 45 degrees, where the reflected light is generated by irradiation of the coated film with light at an incident angle of 0 degrees.

3. The coated titanium oxide powder according to claim 1 or 2, wherein the coated film has a maximum transmittance of transmitted light in a short wavelength region of 580 nm or less in a transmission spectrum at an acceptance angle of 15 degrees and/or a transmittance of 30% or more at a wavelength of 680 nm at an acceptance angle of 15 degrees, where the transmitted light is generated by irradiation of the coated film with light at an incident angle of 0 degrees.

4. The coated titanium oxide powder according to any one of claims 1 to 3, wherein the coated titanium oxide powder has an aspect ratio of the major axis to the minor axis of 1.6 or less.

5. The coated titanium oxide powder according to any one of claims 1 to 4, wherein
the titanium oxide powder is coated with the silicon oxide or hydroxide layer, the aluminum oxide or hydroxide layer, and the iron oxide or hydroxide layer in sequence, and
the iron coating layer is an outermost covering a surface where the silicon coating layer and the aluminum coating layer are mixed.

6. The coated titanium oxide powder according to any one of claims 1 to 5, wherein the surface treatment amount of aluminum is 1 to 6 mass%, the surface treatment amount of silicon is 0.5 to 6 mass%, and the surface treatment amount of iron is 0.5 to 3 mass%, respectively, as oxidized substance equivalent.

7. A cosmetic composition or a dermatological agent comprising the coated titanium oxide powder according to any one of claims 1 to 6.

8. A method for use of the coated titanium oxide powder according to any one of claims 1 to 6, comprising applying the coated titanium oxide powder to the skin to achieve a natural shading effect to the skin.

9. The method for use of the coated titanium oxide powder, according to claim 8, further comprising reducing pallor and yellow dullness of the skin.

## Patentansprüche

1. Beschichtetes Titanoxidpulver, eine Schicht eines Siliziumoxids oder -hydroxids, eine Schicht eines Aluminiumoxids oder -hydroxids und eine Schicht eines Eisenoxids oder -hydroxids umfassend, wobei
das beschichtete Titanoxidpulver einen mittleren Teilchendurchmesser D50 von 0,20 bis 0,45 µm aufweist, gemessen durch Analyse eines transmissionselektronenmikroskopischen Bilds,
das beschichtete Titanoxidpulver einen mittleren Teilchendurchmesser D50 von 0,6 bis 1,2 µm aufweist, gemessen durch Laser-Beugungsstreuung, und
das beschichtete Titanoxidpulver in einer Form eines beschichteten Films ein Verhältnis der Durchlässigkeit von Durchlicht bei einem Eintrittswinkel von 15 Grad zu der Durchlässigkeit von Durchlicht bei einem Eintrittswinkel von 45 Grad von 1,3 oder mehr bei einer Wellenlänge von 430 nm aufweist, und das Verhältnis der Durchlässigkeit von Durchlicht bei einem Eintrittswinkel von 15 Grad zu der Durchlässigkeit von Durchlicht bei einem Eintrittswinkel von 45 Grad bei einer Wellenlänge von 430 nm größer oder gleich dem Verhältnis der Durchlässigkeit von Durchlicht bei einem Eintrittswinkel von 15 Grad zu der Durchlässigkeit von Durchlicht bei einem Eintrittswinkel von 45 Grad bei einer Wellenlänge von 590 nm ist, wobei
der beschichtete Film durch Dispergieren des beschichteten Titanoxids, das einen Gehalt von reinem Titanoxid in einer Menge von netto 10 Masse% und ein Copolymer aus Acrylat und Dimeticon in einer Menge von 20 Masse% in einem flüchtigen Lösemittel enthält, Aufbringen der Dispersion auf eine Glasplatte in einer Dicke von 25 µm und Trocknen der aufgebrachten Schicht hergestellt wird, und das Durchlicht durch Bestrahlung des beschichteten Films mit Licht aus einer CIE-Normlichtquelle D65 eines Spektralphotometersystems mit variablem Winkel bei einem Einfallswinkel von 0 Grad erzeugt wird.

2. Beschichtetes Titanoxidpulver nach Anspruch 1, wobei der beschichtete Film einen maximalen Reflexionsgrad in einem langen Wellenlängenbereich von 450 nm oder mehr in einem Reflexionsspektrum bei einem Eintrittswinkel von 45 Grad aufweist, wobei das reflektierte Licht durch Bestrahlung des beschichteten Films mit Licht bei einem Einfallswinkel von 0 Grad erzeugt wird.

3. Beschichtetes Titanoxidpulver nach Anspruch 1 oder 2, wobei der beschichtete Film eine maximale Durchlässigkeit von Durchlicht in einem kurzen Wellenlängenbereich von 580 nm oder weniger in einem Transmissionsspektrum bei einem Eintrittswinkel von 15 Grad und/oder einer Durchlässigkeit von 30 % oder mehr bei einer Wellenlänge von 680 nm bei einem Eintrittswinkel von 15 Grad aufweist, wobei das Durchlicht durch Bestrahlung des beschichteten Films mit Licht bei einem Einfallswinkel von 0 Grad erzeugt wird.

4. Beschichtetes Titanoxidpulver nach einem der Ansprüche 1 bis 3, wobei das beschichtete Titanoxidpulver ein Aspektverhältnis der Hauptachse zu der Nebenachse von 1,6 oder weniger aufweist.

5. Beschichtetes Titanoxidpulver nach einem der Ansprüche 1 bis 4, wobei
das Titanoxidpulver der Reihe nach mit der Siliziumoxidschicht oder Siliziumhydroxidschicht, der Aluminiumoxidschicht oder Aluminiumhydroxidschicht und der Eisenoxidschicht oder Eisenhydroxidschicht beschichtet ist und
die Eisenschicht eine äußerste Bedeckung einer Oberfläche ist, wobei die Siliziumschicht und die Aluminiumschicht gemischt sind.

6. Beschichtetes Titanoxidpulver nach einem der Ansprüche 1 bis 5, wobei die Oberflächenbehandlungsmenge von Aluminium 1 bis 6 Masse% ist, die Oberflächenbehandlungsmenge von Silizium 0,5 bis 6 Masse% ist bzw. die Oberflächenbehandlungsmenge von Eisen 0,5 bis 3 Masse% als Äquivalent der oxidierten Substanz ist.

7. Kosmetische Zusammensetzung oder dermatologischer Wirkstoff, das beschichtete Titanoxidpulver nach einem der Ansprüche 1 bis 6 umfassend.

8. Verfahren zur Verwendung des beschichteten Titanoxidpulvers nach einem der Ansprüche 1 bis 6, Aufbringen des beschichteten Titanoxidpulvers auf die Haut umfassend, um eine natürliche Schattenwirkung auf der Haut zu erzielen.

9. Verfahren zur Verwendung des beschichteten Titanoxidpulvers nach Anspruch 8, weiterhin Reduzieren von Blässe und Gelbmattheit der Haut umfassend.

## Revendications

1. Poudre d'oxide de titane enrobée, comprenant une couche d'enrobage d'un oxyde ou hydroxyde de silicium, une couche d'enrobage d'un oxyde ou hydroxyde d'aluminium, et une couche d'enrobage d'un oxyde ou hydroxyde de fer, dans laquelle
la poudre d'oxide de titane enrobée présente un diamètre moyen des particules D50 de 0,20 à 0,45 µm mesuré par analyse d'une image microscopique électronique en transmission,
la poudre d'oxide de titane enrobée présente un diamètre moyen des particules D50 de 0,6 à 1,2 µm mesuré par dispersion par diffraction laser, et
la poudre d'oxide de titane enrobée sous une forme d'un film enrobé présente un rapport de la transmittance de lumière transmise à un angle d'acceptance de 15 degrés par rapport à la transmittance de lumière transmise à un angle d'acceptance de 45 degrés de 1,3 ou plus à une longueur d'onde de 430 nm, et le rapport de la transmittance de lumière transmise à un angle d'acceptance de 15 degrés par rapport à la transmittance de lumière transmise à un angle d'acceptance de 45 degrés à une longueur d'onde de 430 nm est égal ou supérieur au rapport de la transmittance de lumière transmise à un angle d'acceptance de 15 degrés par rapport à la transmittance de lumière transmise à un angle d'acceptance de 45 degrés à une longueur d'onde de 590 nm, où
le film enrobé est préparé en dispersant l'oxyde de titane enrobé comprenant une teneur en oxyde de titane pur en une quantité de 10 % net en masse et un copolymère d'acrylate et de diméthicone en une quantité de 20 % en masse dans un solvant volatil, appliquant la dispersion sur une plaque de verre en une épaisseur de 25 µm, et séchant la couche appliquée, et la lumière transmise est générée par irradiation du film enrobé avec de la lumière provenant d'une source de lumière D65, conforme à la norme CIE, d'un système spectrophotométrique angle à variable à un angle d'incidence de 0 degré.

2. Poudre d'oxide de titane enrobée selon la revendication 1, dans laquelle le film enrobé présente une réflectance maximum dans une région de longue longueur d'onde de 450 nm ou plus dans un spectre de réflexion à un angle d'acceptance de 45 degrés, où la lumière réfléchie est générée par irradiation du film enrobé avec de la lumière à un angle d'incidence de 0 degré.

3. Poudre d'oxide de titane enrobée selon la revendication 1 ou 2, dans laquelle le film enrobé présente une transmittance maximum de lumière transmise dans une région de courte longueur d'onde de 580 nm ou moins dans un spectre de transmission à un angle d'acceptance de 15 degrés et/ou une transmittance de 30 % ou plus à une longueur d'onde de 680 nm à un angle d'acceptance de 15 degrés, où la lumière transmise est générée par irradiation du film enrobé avec de la lumière à un angle d'incidence de 0 degré.

4. Poudre d'oxide de titane enrobée selon l'une quelconque des revendications 1 à 3, dans laquelle la poudre d'oxide de titane enrobée présente un rapport d'aspect de l'axe majeur par rapport à l'axe mineur de 1,6 ou moins.

5. Poudre d'oxide de titane enrobée selon l'une quelconque des revendications 1 à 4, dans laquelle
la poudre d'oxyde de titane est enrobée avec la couche d'oxyde ou d'hydroxyde de silicium, la couche d'oxyde ou d'hydroxyde d'aluminium, et la couche d'oxyde ou d'hydroxyde de fer en séquence, et
la couche d'enrobage en fer est une couche la plus extérieure couvrant une surface où la couche d'enrobage en silicium et la couche d'enrobage en aluminium sont mélangées.

6. Poudre d'oxide de titane enrobée selon l'une quelconque des revendications 1 à 5, dans laquelle la quantité de traitement de surface de l'aluminium est de 1 à 6 % en masse, la quantité de traitement de surface du silicium est de 0,5 à 6 % en masse, et la quantité de traitement de surface du fer est de 0,5 à 3 % en masse, respectivement, en tant qu'équivalent de substance oxydée.

7. Composition cosmétique ou agent dermatologique, comprenant la poudre d'oxide de titane enrobée selon l'une quelconque des revendications 1 à 6.

8. Procédé pour utilisation de la poudre d'oxide de titane enrobée, selon l'une quelconque des revendications 1 à 6, comprenant l'application de la poudre d'oxide de titane enrobée sur la peau pour obtenir un effet d'ombrage naturel sur la peau.

9. Procédé pour utilisation de la poudre d'oxide de titane enrobée, selon la revendication 8, comprenant en outre la réduction de pâleur et de teint mat jaunâtre de la peau.
